# EUROPEAN PATENT APPLICATION

(11) **EP 4 112 126 A1**
(43) Date of publication of application: **04.01.2023**
(21) Application number: 20922390.8
(22) Date of filing: 10.12.2020
(51) Int. Cl.: A61Q 1/00, A61Q 15/00, A61Q 17/04, A61Q 19/00, C08L 83/04, A61Q 5/00, A61K 8/06, A61K 8/89

(54) **COSMETIC**

(30) Priority: 27.02.2020 JP 2020031280
(71) Applicant: Shin-Etsu Chemical Co., Ltd., Tokyo 100-0005 (JP)
(72) Inventor: ABE Takuya, Annaka-shi, Gunma 379-0224 (JP); KAMEI Masanao, Annaka-shi, Gunma 379-0224 (JP)
(74) Representative: Sonnenhauser, Thomas Martin
(86) International application number: PCT/JP2020/045949
(87) International publication number: WO 2021/171735

(57) **Abstract**

The present invention is a cosmetic containing a silicone resin whose constitutional units consist of general formulae (A) (R¹₃SiO_{1/2})a, (B) (R²₂SiO_{2/2})b, (C) (R³SiO_{3/2})c, and (D) (SiO_{4/2})d, where R¹, R², and R³ each independently represent a group selected from an alkyl group having 1 to 8 carbon atoms, an aryl group having 6 to 12 carbon atoms, and a fluorine-substituted alkyl group having 1 to 8 carbon atoms, "a" has a value of 0.2 to 0.5, "b" has a value of 0.0 to 0.1, "c" has a value of 0.2 to 0.6, "d" has a value of 0.2 to 0.5, a+b+c+d has a value of 1.0, a/(c+d) has a value of 0.3 to 0.9, and c/d has a value of 0.4 to 3.0, the silicone resin having a weight-average molecular weight of more than 20000 and 100000 or less. An object of the present invention is to provide accordingly a cosmetic that has no stickiness upon application, has excellent cosmetic durability, and can prevent adhesion to clothes or the like (secondary adhesion).

## Description

### TECHNICAL FIELD

The present invention relates to a cosmetic.

### BACKGROUND ART

Conventionally, silicone resin has film-formation ability and is excellent in water resistance, perspiration resistance, and sebum resistance, and is therefore used as a raw material for cosmetics such as make-up cosmetics including foundation, lipstick, eye shadow, mascara, etc., ultraviolet protection cosmetics, and hair cosmetics. For example, the following are known: a skin cosmetic containing an organic silicone resin composed of units having an average formula of RₙSiO_{4-n/2} and a volatile hydrocarbon oil (Patent Document 1); a skin cosmetic containing a resin composed of an R₃SiO_{1/2} unit and an SiO_{4/2} unit and a volatile silicone oil (Patent Document 2); a sunscreen cosmetic containing a silicone resin having two or more kinds of an R₂SiO_{2/2} unit, an RSiO_{3/2} unit, and an SiO_{4/2} unit and optionally having the terminals blocked with R₃SiO_{1/2} units, a volatile oil agent, and an ultraviolet absorber and/or ultraviolet scattering agent (Patent Document 3); a skin cosmetic containing 1 to 70 mass% of an organic silicone resin, 70 mol% or more of the organic silicone resin consisting of an R₃SiO_{1/2} unit and an SiO_{4/2} unit, and the molar ratio of an R₃SiO_{1/2} unit to an SiO_{4/2} unit being from 0.5/1 to 1.5/1 (Patent Document 4); and so forth. These disclose that a film excellent in water resistance can be obtained, but do not disclose details regarding stiffness or feel of the film owing to the composition of the silicone resin.

In these known techniques, an SiO_{4/2} unit is contained, so that there is a problem that a stretched feeling is often given due to the stiffness of the film. Accordingly, to improve stiffness and brittleness of the film, the following are proposed: a cosmetic containing a silicone resin composed of 50 to 99 mol% of an RSiO_{3/2} unit and 1 to 50 mol% of an R₃SiO_{1/2} unit (Patent Document 5); a cosmetic containing a silicone resin, the molar ratio of (A) an R₃SiO_{1/2} unit to (B) an RSiO_{3/2} unit being 1:4.7 to 1:7, and the silicone resin having a softening point of 50 to 110°C and a weight-average molecular weight of 8000 to 400000 (Patent Document 6).

In these proposals, the crosslinking components of the silicone resin are constituted solely of RSiO_{3/2} units, and therefore, there are few crosslinking points. Accordingly, in order to improve water resistance, perspiration resistance, resistance to friction, and so forth more than before, the weight-average molecular weight needs to be 8000 or more. Moreover, since silanol groups remaining in the silicone resin exhibit thermosetting properties due to heating at a high temperature over a long period, the cosmetics are manufactured in a two-stage process including blocking with R₃SiCl or the like in order to reduce the silanol groups.

In Patent Document 7, a cosmetic excellent in cosmetic durability and secondary adhesion is obtained by blending a silicone resin, the molar ratio of an RSiO_{3/2} unit and an SiO_{4/2} unit being 0.8:1.0 to 2.5:1.0, and the silicone resin having a weight-average molecular weight of 1500 to 20000. However, silicone resin has high compatibility with hydrocarbon oil, ester oil, triglyceride, etc., which are components of sebum, and physical strength of a cosmetic film is degraded over time, so that cosmetic durability is degraded. Therefore, further improvement of cosmetic durability is required. Furthermore, since more RSiO_{3/2} units are contained compared with a resin containing R₃SiO_{1/2} units and SiO_{4/2} units in the same molecular weight, strength of a film is low, and issues are found in abrasion resistance.

### CITATION LIST

### PATENT LITERATURE

Patent Document 1: JP H04-59284 B2
Patent Document 2: JP H06-15448 B2
Patent Document 3: JP S62-234012 A
Patent Document 4: JP S62-298511 A
Patent Document 5: JP 2636538 B2
Patent Document 6: JP 5705538 B2
Patent Document 7: JP 2018-2643 A

### SUMMARY OF INVENTION

### TECHNICAL PROBLEM

The present invention has been made in view of the above-described problems, and an object thereof is to provide a cosmetic that has no stickiness upon application, has smooth spreadability, excellent feeling on use, good water resistance, and excellent cosmetic durability owing to good adhesion to skin, can prevent adhesion to clothes or the like (secondary adhesion), and has excellent abrasion resistance.

### SOLUTION TO PROBLEM

To achieve the object, the present invention provides a cosmetic comprising a silicone resin whose constitutional units consist of the following general formulae (A), (B), (C), and (D):

(A) (R¹₃SiO_{1/2}) a;

(B) (R²₂SiO_{2/2})b;

(C) (R³SiO_{3/2})c;

and

(D) (SiO_{4/2})d,

wherein R¹, R², and R³ each independently represent a group selected from an alkyl group having 1 to 8 carbon atoms, an aryl group having 6 to 12 carbon atoms, and a fluorine-substituted alkyl group having 1 to 8 carbon atoms,
"a" has a value of 0.2 to 0.5,
"b" has a value of 0.0 to 0.1,
"c" has a value of 0.2 to 0.6,
"d" has a value of 0.2 to 0.5,
a+b+c+d has a value of 1.0, a/(c+d) has a value of 0.3 to 0.9, and c/d has a value of 0.4 to 3.0,
   the silicone resin having a weight-average molecular weight of more than 20000 and 100000 or less.

Such a cosmetic has no stickiness upon application, has smooth spreadability, excellent feeling on use, good water resistance, and excellent cosmetic durability owing to good adhesion to skin, can prevent adhesion to clothes or the like (secondary adhesion), and has excellent abrasion resistance.

Furthermore, the cosmetic preferably contains the silicone resin in an amount of 0.1 to 40 mass% based on a total mass of the cosmetic.

When the contained amount is as described, the cosmetic has particularly excellent cosmetic durability and so forth.

Furthermore, the cosmetic preferably further comprises an oil agent selected from a silicone oil, a hydrocarbon oil, an ester oil, and a glyceride oil.

As described, the inventive cosmetic can contain various oil agents according to the object of the cosmetic.

Furthermore, the cosmetic preferably further comprises a surfactant.

When a surfactant is contained as described, an emulsion is obtained more easily.

In this case, the surfactant is preferably: a linear or branched organopolysiloxane whose hydrophilic group is polyoxyalkylene or polyglycerin; or an alkyl-co-modified organopolysiloxane thereof.

These surfactants are particularly suitable as the surfactant to be contained in the inventive cosmetic.

Furthermore, the cosmetic preferably further comprises a composition composed of crosslinked organopolysiloxane polymer and a liquid oil agent.

As described, the inventive cosmetic can contain the above-described composition according to the object of the cosmetic.

In this case, the crosslinked organopolysiloxane polymer may have a polyether group and/or a polyglycerin group.

When the inventive cosmetic contains the above-described composition, the crosslinked organopolysiloxane polymer may have the above-described group.

Furthermore, the cosmetic preferably further comprises a silicone film former selected from a group consisting of: other than the silicone resin, a silicone resin composed of components selected from R₃SiO_{1/2}, R₂SiO_{2/2}, RSiO_{3/2}, and SiO_{4/2}, wherein R is the same as the R¹; a linear acrylic-silicone graft copolymer; and a linear acrylic-silicone block copolymer.

When a silicone film former is contained as described, the film-formation ability of the inventive cosmetic can be further enhanced.

Furthermore, the cosmetic preferably further comprises a powder.

The inventive cosmetic does not undergo change by agglomeration of powder, etc., and is excellent in dispersion stability of powder even when a powder is contained.

Furthermore, the cosmetic preferably further comprises an ultraviolet light absorbing component.

Such a cosmetic can absorb ultraviolet light effectively.

Furthermore, the cosmetic is preferably one of a water-based, oil-based, or emulsion-based skin care cosmetic, hair cosmetic, antiperspirant, make-up cosmetic, or ultraviolet protection cosmetic.

As described, the inventive cosmetic can adopt various forms in accordance with each type of cosmetic.

### ADVANTAGEOUS EFFECTS OF INVENTION

The inventive cosmetic has no stickiness upon application, has smooth spreadability, excellent feeling on use, good water resistance, and excellent cosmetic durability owing to good adhesion to skin, can prevent adhesion to clothes or the like (secondary adhesion), and has excellent abrasion resistance.

### DESCRIPTION OF EMBODIMENTS

Hereinafter, the present invention will be described in more detail.

As described above, there are demands for a cosmetic that has no stickiness upon application, has smooth spreadability, excellent feeling on use, good water resistance, and excellent cosmetic durability owing to good adhesion to skin, can prevent adhesion to clothes or the like (secondary adhesion), and has excellent abrasion resistance.

Silicone resins that can be contained in a cosmetic can be designed to have various forms from liquid to solid by adjusting the crosslinking degree and molecular weight by a combination of monofunctional R₃SiO_{1/2} units, difunctional R₂SiO_{2/2} units, trifunctional RSiO_{3/2} units, and tetrafunctional SiO_{4/2} units. On this occasion, the present inventors have studied the composition of each unit in detail, and succeeded in finding a silicone resin that contains tetrafunctional a SiO_{4/2} unit in a composition within a specific range, makes it possible to obtain a film having no stickiness, can also be manufactured easily in one stage regarding the manufacturing method, and has good stability over time. Furthermore, by designing the silicone resin to have a weight-average molecular weight of more than 20000 and 100000 or less, a film having more excellent abrasion resistance can be obtained compared with a case where the molecular weight is 20000 or less. Furthermore, the present inventors have found out that when this silicone resin is contained in a cosmetic, it is possible to provide a cosmetic that has no stickiness upon application, has smooth spreadability, excellent feeling on use, good water resistance, and excellent cosmetic durability owing to good adhesion to skin, can prevent adhesion to clothes or the like (secondary adhesion), and has excellent abrasion resistance, and arrived at the present invention.

That is, the present invention is a cosmetic comprising a silicone resin whose constitutional units consist of the following general formulae (A), (B), (C), and (D):

(A) (R¹₃SiO_{1/2})a;

(B) (R²₂SiO_{2/2}) b;

(C) (R³SiO_{3/2})c;

and

(D) (SiO_{4/2})d,

wherein R¹, R², and R³ each independently represent a group selected from an alkyl group having 1 to 8 carbon atoms, an aryl group having 6 to 12 carbon atoms, and a fluorine-substituted alkyl group having 1 to 8 carbon atoms,
"a" has a value of 0.2 to 0.5,
"b" has a value of 0.0 to 0.1,
"c" has a value of 0.2 to 0.6,
"d" has a value of 0.2 to 0.5,
a+b+c+d has a value of 1.0, a/(c+d) has a value of 0.3 to 0.9, and c/d has a value of 0.4 to 3.0,
   the silicone resin having a weight-average molecular weight of more than 20000 and 100000 or less.

Hereinafter, embodiments of the present invention will be described specifically, but the present invention is not limited thereto.

The inventive cosmetic contains a silicone resin (hereinafter, also referred to as silicone resin (I)) whose constitutional units consist of the following general formulae (A), (B), (C), and (D):

(A) (R¹₃SiO_{1/2}) a;

(B) (R²₂SiO_{2/2}) b;

(C) (R³SiO_{3/2}) c;

and

(D) (SiO_{4/2})d,

the silicone resin having a weight-average molecular weight of more than 20000 and 100000 or less.

R¹, R², and R³ in the silicone resin (I) each independently represent a group selected from an alkyl group having 1 to 8 carbon atoms, an aryl group having 6 to 12 carbon atoms, and a fluorine-substituted alkyl group having 1 to 8 carbon atoms. Specifically, examples of the alkyl group having 1 to 8 carbon atoms include a methyl group, an ethyl group, a propyl group, a butyl group, a pentyl group, a hexyl group, a heptyl group, and an octyl group. Examples of the fluorine-substituted alkyl group having 1 to 8 carbon atoms include a trifluoropropyl group, etc. Examples of the aryl group having 6 to 12 carbon atoms include a phenyl group, a tolyl group, etc. An alkyl group having 1 to 3 carbon atoms or a phenyl group is preferable, and the group is more preferably selected from a methyl group and a phenyl group. Furthermore, 50% or more of the R¹, R², and R³ are preferably methyl groups, and most preferably, 70% or more of the R¹, R², and R³ are methyl groups.

a+b+c+d has a value of 1.0. "a" has a value of 0.2 to 0.5. If "a" is less than 0.2, the cosmetic film becomes hard and brittle, and if more than 0.5, the cosmetic film becomes sticky or liquid. "a" is more preferably 0.25 to 0.40. "b" has a value of 0.0 to 0.1. If "b" is more than 0.1, the cosmetic film becomes sticky. "b" is more preferably 0.0 to 0.05. "c" has a value of 0.2 to 0.6. If "c" is less than 0.2, the cosmetic film becomes hard and brittle, and if more than 0.6, the cosmetic film becomes sticky. "c" is more preferably 0.2 to 0.5. "d" has a value of 0.2 to 0.5. If "d" is less than 0.2, the cosmetic film becomes sticky, and if more than 0.5, the cosmetic film becomes hard and brittle. "d" is more preferably 0.25 to 0.40.

a/(c+d) has a value of 0.3 to 0.9. If a/(c+d) is less than 0.3, the cosmetic film becomes hard, and if more than 0.9, the cosmetic film becomes sticky. a/(c+d) is more preferably 0.35 to 0.85. c/d has a value of 0.4 to 3.0. If c/d is less than 0.4, the cosmetic film becomes hard, and if more than 3.0, the cosmetic film becomes sticky. c/d is preferably 0.5 to 2.3.

The silicone resin (I) has a weight-average molecular weight of more than 20000 and 100000 or less. The weight-average molecular weight is more preferably more than 20000 and 50000 or less. This silicone resin (I) is capable of forming a film having no stickiness. In particular, the silicone resin (I) contains tetrafunctional SiO_{4/2} units in a composition within a specific range, so that a film having no stickiness can be formed, and stability over time, water resistance, and so forth are favorable. In addition a film having excellent abrasion resistance can be obtained compared with a silicone resin having a weight-average molecular weight of 20000 or less. Accordingly, the inventive cosmetic, containing this silicone resin, has no stickiness upon application, has smooth spreadability, excellent feeling on use, good water resistance, and excellent cosmetic durability owing to good adhesion to skin, can prevent adhesion to clothes or the like (secondary adhesion), and has excellent abrasion resistance. Note that the weight-average molecular weight can be determined as a weight-average molecular weight in terms of polystyrene in a gel permeation chromatography (GPC) analysis.

The silicone resin (I) is usually solid, and therefore, may be a ground solid, or may be a solution diluted with an oil agent that can be used in cosmetics.

The silicone resin (I) can be manufactured by a known method. That is, the silicone resin (I) can be manufactured by performing a hydrolysis condensation reaction between organosilicon compounds shown by the following general formulae (1) and (2) and compounds shown by the following formula (3), the following formula (4), and the following formula (5). Note that regarding the compounds shown by the following formulae (3) to (5), partial hydrolysis products thereof may be used instead of the compounds, or partial hydrolysis products thereof may be used in addition to the compounds.

R¹₃SiOSiR¹₃ (1)

R1₃SiOR⁴ (2)

R²₂Si (OR⁴) ₂ (3)

R³Si (OR⁴)₃ (4)

Si (OR⁴)₄ (5)

R¹, R², and R³ are as defined above, and R⁴ represents either a hydrogen atom or an alkyl group having 1 to 4 carbon atoms.

This hydrolysis condensation reaction is preferably performed under acidic condition, and an acidic substance is added as a catalyst. Examples of the acidic substance include hydrochloric acid, sulfuric acid, p-toluenesulfonic acid, methanesulfonic acid, trifluoromethanesulfonic acid, phosphoric acid, acetic acid, citric acid, etc. The amount of the acidic substance to be used may be a small amount, and is preferably 0.001 to 10 mass% of the entire system.

In addition, a solvent can be used when performing the hydrolysis condensation reaction. Examples of the solvent include hydrocarbon-based solvents such as toluene, xylene, and isoparaffin, ether-based solvents such as tetrahydrofuran, and alcohol-based solvents such as methanol, ethanol, (iso)propyl alcohol, and butanol. The solvent is preferably ethanol or (iso)propyl alcohol.

Examples of a specific method for the hydrolysis condensation reaction include the method shown below. Firstly, a solvent and a hydrolysable raw material are charged into a reactor, an acid is added thereto, and water is dropped thereto while stirring. The temperature when dropping the water is preferably 0 to 80°C, particularly preferably 0 to 50°C, and the amount of the water to be dropped in is preferably within the range of 0.6 to 2.0 by molar ratio relative to the hydrolysable group. After dropping the water in, the mixture is heated at 50 to 150°C, preferably 80 to 120°C for 2 to 8 hours to perform a hydrolysis condensation reaction. After the hydrolysis, acid is removed. To remove the acid, it is possible to employ a method of neutralization with alkali metal carbonate, alkali metal hydrogencarbonate, alkali metal hydroxide, etc. The acid may also be removed in a step of washing with water. After neutralizing the acid, an oil agent that can be used in cosmetics is added, and only the generated alcohols and excess water are removed by heating under normal pressure or reduced pressure. Thus, a silicone resin solution can be obtained. In addition, the oil agent that can be used in cosmetics may also be added during the hydrolysis condensation reaction.

Since the silicone resin (I) is usually solid, it is preferable to form a solution by diluting with an oil agent that can be used in cosmetics. As such a diluent, it is possible to use a volatile siloxane compound such as octamethyltrisiloxane, decamethyltetrasiloxane, decamethylcyclopentasiloxane, and tristrimethylsiloxy methylsilane, and volatile hydrocarbon compounds such as isododecane.

In the inventive cosmetic, the contained amount of the silicone resin (I) is preferably within a range of 0.1 to 40 mass% based on the total mass of the cosmetic. When the contained amount is as described, a cosmetic excellent in cosmetic durability, etc. in particular can be achieved. The amount to be added more preferably has a range of 0.5 to 20 mass%.

The inventive cosmetic can contain one or more oil agents according to the object. An oil agent in any form of solid, semi-solid, or liquid can be used as long as it is used in usual cosmetics. For example, it is possible to use natural vegetable and animal fats and oils, semi-synthetic fats and oils, hydrocarbon oils, higher fatty acids, higher alcohols, ester oils, glyceride oils, commonly used silicone oils, fluorinated oil agents, and the like.

Examples of the natural animal and vegetable oils and fats and semi-synthetic oils and fats include avocado oil, linseed oil, almond oil, insect wax, perilla oil, olive oil, cacao butter, kapok wax, kaya oil, carnauba wax, liver oil, candelilla wax, purified candelilla wax, beef tallow, neats foot fat, beef bone fat, hardened beef tallow, apricot kernel oil, whale wax, hardened oil, wheat germ oil, sesame oil, rice germ oil, rice bran oil, sugarcane wax, Camellia sasanqua oil, safflower oil, shea butter, Chinese tung oil, cinnamon oil, jojoba wax, squalane, squalene, shellac wax, turtle oil, soybean oil, tea seed oil, camellia oil, evening primrose oil, corn oil, lard, rapeseed oil, Japanese tung oil, bran wax, germ oil, horse fat, persic oil, palm oil, palm kernel oil, castor oil, hardened castor oil, castor oil fatty acid methyl ester, sunflower oil, grape oil, bayberry wax, jojoba oil, macadamia nut oil, bees wax, mink oil, meadowfoam oil, cottonseed oil, cotton wax, Japan wax, Japan wax kernel oil, montan wax, coconut oil, hardened coconut oil, tri-coconut oil fatty acid glyceride, mutton tallow, peanut oil, lanolin, liquid lanolin, reduced lanolin, lanolin alcohol, hard lanolin, lanolin acetate, acetylated lanolin alcohol, lanolin fatty acid isopropyl, POE lanolin alcohol ether, POE lanolin alcohol acetate, lanolin fatty acid polyethylene glycol, POE hydrogenated lanolin alcohol ether, egg yolk oil, etc.

Provided that POE means polyoxyethylene (the same applies hereinafter).

Examples of the hydrocarbon oils include linear, branched, and further volatile hydrocarbon oils, etc., and specific examples include ozokerite, α-olefin oligomer, light isoparaffin, isododecane, isohexadecane, light liquid isoparaffin, squalane, synthetic squalane, vegetable squalane, squalene, ceresin, paraffin, paraffin wax, polyethylene wax, polyethylene·polypropylene wax, an (ethylene/propylene/styrene) copolymer, a (butylene/propylene/styrene) copolymer, liquid paraffin, liquid isoparaffin, pristane, polyisobutylene, hydrogenated polyisobutene, microcrystalline wax, vaseline, etc.

Examples of the higher fatty acid include lauric acid, myristic acid, palmitic acid, stearic acid, behenic acid, undecylenic acid, oleic acid, linoleic acid, linolenic acid, arachidonic acid, eicosapentaenoic acid (EPA), docosahexaenoic acid (DHA), isostearic acid, 12-hydroxystearic acid, etc.

Examples of the higher alcohols include lauryl alcohol, myristyl alcohol, palmityl alcohol, stearyl alcohol, behenyl alcohol, hexadecyl alcohol, oleyl alcohol, isostearyl alcohol, hexyldodecanol, octyldodecanol, cetostearyl alcohol, 2-decyltetradecynol, cholesterol, phytosterol, POE cholesterol ether, monostearyl glycerin ether (batyl alcohol), monooleyl glyceryl ether (selacyl alcohol), etc.

Examples of the ester oils include diisobutyl adipate, 2-hexyldecyl adipate, di-2-heptylundecyl adipate, N-alkylglycol monoisostearate, isocetyl isostearate, trimethylolpropane triisostearate, ethylene glycol di-2-ethylhexanoate, cetyl 2-ethylhexanoate, trimethylolpropane tri-2-ethylhexanoate, pentaerythritol tetra-2-ethylhexanoate, cetyl octanoate, octyldodecyl gum ester, oleyl oleate, octyldodecyl oleate, decyl oleate, neopentyl glycol dioctanoate, neopentyl glycol dicaprate, triethyl citrate, 2-ethylhexyl succinate, amyl acetate, ethyl acetate, butyl acetate, isocetyl stearate, butyl stearate, diisopropyl sebacate, di-2-ethylhexyl sebacate, cetyl lactate, myristyl lactate, isononyl isononanate, isotridecyl isononanate, isopropyl palmitate, 2-ethylhexyl palmitate, 2-hexyldecyl palmitate, 2-heptylundecyl palmitate, cholesteryl 12-hydroxystearate, dipentaerythritol fatty acid ester, isopropyl myristate, octyldodecyl myristate, 2-hexyldecyl myristate, myristyl myristate, hexyldecyl dimethyloctanoate, ethyl laurate, hexyl laurate, N-lauroyl-L-glutamic acid-2-octyldodecyl ester, isopropyl lauroyl sarcosinate, diisostearyl malate, etc.

Examples of the glyceride oil include acetoglyceryl, glyceryl triisooctanoate, glyceryl triisostearate, glyceryl triisopalmitate, glyceryl tribehenate, glyceryl monostearate, glyceryl di-2-heptylundecanoate, glyceryl trimyristate, diglyceryl isostearate/myristate, etc.

Examples of the silicone oils include low viscous to high viscous linear or branched organopolysiloxanes such as dimethyl polysiloxane, tristrimethylsiloxy methylsilane, caprylyl methicone, phenyl trimethicone, tetrakistrimethylsiloxysilane, methylphenylpolysiloxane, methylhexylpolysiloxane, methyl hydrogen polysiloxane, and dimethylsiloxane/methylphenylsiloxane copolymers; cyclic organopolysiloxanes such as octamethyl cyclotetrasiloxane, decamethyl cyclopentasiloxane, dodecamethyl cyclohexasiloxane, tetramethyl tetrahydrogen cyclotetrasiloxane, and tetramethyltetraphenyl cyclotetrasiloxane; silicone rubbers such as amino-modified organopolysiloxanes, pyrrolidone-modified organopolysiloxanes, pyrrolidone carboxylate-modified organopolysiloxanes, gum dimethyl polysiloxanes with high polymerization degree, gum amino-modified organopolysiloxanes, and gum dimethylsiloxane/methylphenylsiloxane copolymers; silicone gum and rubber cyclic organopolysiloxane solutions, higher alkoxy-modified silicone such as stearoxysilicone, higher fatty acid-modified silicones, alkyl-modified silicones, long chain alkyl-modified silicones, amino acid-modified silicones, fluorinemodified silicones, and the like.

Examples of the fluorinated oil agents include perfluoropolyether, perfluorodecalin, perfluorooctane, etc.

The inventive cosmetic can further contain an oil agent selected from a silicone oil, a hydrocarbon oil, an ester oil, and a glyceride oil. The amount of these oil agents to be contained may vary depending on the agent system, but is preferably in the range of 1 to 98 mass% based on the entire cosmetic.

The inventive cosmetic can also contain water depending on the purpose. The contained amount of the water may vary depending on the agent system, but is preferably in the range of 1 to 95 mass% based on the entire cosmetic.

In the inventive cosmetic, one or more kinds of a lower alcohol having 2 to 5 carbon atoms and a polyhydric alcohol having 2 to 10 carbon atoms may be used depending on the purpose. Examples of the alcohol include a lower alcohol such as ethanol and isopropanol; a sugar alcohol such as sorbitol and maltose; a sterol such as cholesterol, sitosterol, phytosterol and lanosterol; and a polyhydric alcohol such as butylene glycol, propylene glycol, dibutylene glycol, and pentylene glycol. As the amount to be contained, a range of 0.1 to 98 mass% based on the entire cosmetic is suitable.

In the inventive cosmetic, a water-soluble or water-swellable polymer can be used depending on the purpose. Examples thereof include plant polymers such as an Arabia gum, tragacanth, galactan, a carob gum, a guar gum, a karaya gum, carrageenan, pectin, agar, quince seed (marmelo), starch (rice, corn, potato, wheat, and so on), an algae colloid, a trant gum, and a locust bean gum; microbial polymers such as a xanthan gum, dextran, succinoglucan, and pullulan; animal polymers such as collagen, casein, albumin, and gelatin; starch polymers such as carboxymethyl starch and methyl hydroxypropyl starch; cellulose polymers such as methyl cellulose, ethyl cellulose, methyl hydroxypropyl cellulose, carboxymethyl cellulose, hydroxymethyl cellulose, hydroxypropyl cellulose, nitrocellulose, sodium cellulose sulfate, sodium carboxymethyl cellulose, crystalline cellulose, and cellulose powder; alginic acid polymers such as sodium alginate and propylene glycol alginate ester; vinyl polymers such as polyvinyl methyl ether and carboxy vinyl polymer; a polyoxyethylene polymer; polyoxyethylene polyoxypropylene copolymer polymers; acryl polymers such as sodium polyacrylate, polyethyl acrylate, polyacrylamide, and an acryloyldimethyl taurate salt copolymer; other synthetic water-soluble polymers such as polyethyleneimine and a cationic polymer; inorganic water-soluble polymers such as a bentonite, aluminum magnesium silicate, montmorillonite, beidellite, nontronite, saponite, hectorite, and anhydrous silicic acid; and the like.

In particular, one or more water-soluble or water-swellable polymers selected from plant polymers, microbial polymers, animal polymers, starch polymers, cellulose polymers, alginic acid polymers, polyoxyethylene polyoxypropylene copolymer polymers, acryl polymers, and inorganic water-soluble polymers are preferably used. As the amount to be contained when using a water-soluble or water-swellable polymer, a range of 0.1 to 25 mass% based on the entire cosmetic is suitable.

In the inventive cosmetic, one or more powders can also be used depending on the purpose. As the powder, any of the materials can also be used as long as it is used in the usual cosmetics, regardless of its shape (spherical, needle, plate, etc.), particle diameter (fumed, fine particles, pigment grade, etc.), or particulate structure (porous, nonporous, etc.), and examples of the powder include powders selected from an inorganic powder, an organic powder, a surfactant metal salt powder, a colored pigment, a pearl pigment, a metal powder pigment, a tar pigment, a natural pigment, etc. The inventive cosmetic does not undergo change by agglomeration of powder, etc., and is excellent in dispersion stability of powder even when a powder is contained.

Specific examples of the inorganic powder include titanium oxide, zirconium oxide, zinc oxide, cerium oxide, magnesium oxide, barium sulfate, calcium sulfate, magnesium sulfate, calcium carbonate, magnesium carbonate, talc, mica, kaolin, sericite, muscovite, synthetic mica, phlogopite, lepidolite, biotite, lithia mica, silicic acid, silicic anhydride, aluminum silicate, magnesium silicate, magnesium aluminum silicate, calcium silicate, barium silicate, strontium silicate, metal tungstate, hydroxyapatite, vermiculite, Higilite, bentonite, montmorillonite, hectorite, zeolite, ceramic powder, dibasic calcium phosphate, alumina, aluminum hydroxide, boron nitride, boron nitride, silica, etc.

Examples of the organic powder include polyamide powder, polyester powder, polyethylene powder, polypropylene powder, polystyrene powder, polyurethane, benzoguanamine powder, polymethylbenzoguanamine powder, tetrafluoroethylene powder, polymethyl methacrylate powder, cellulose, silk powder, Nylon powder, 12 Nylon, 6 Nylon, silicone powder, styrene · acrylic acid copolymer, divinylbenzene·styrene copolymer, vinyl resin, urea resin, phenol resin, fluorine resin, silicon resin, acrylic resin, melamine resin, epoxy resin, polycarbonate resin, microcrystalline fiber powder, starch powder, lauroyl lysine, etc.

Examples of the surfactant metal salt powder (metallic soap) include zinc stearate, aluminum stearate, calcium stearate, magnesium stearate, zinc myristate, magnesium myristate, zinc cetyl phosphate, calcium cetyl phosphate, zinc sodium cetyl phosphate, etc.

Examples of the colored pigment include inorganic red pigments such as iron oxide, iron hydroxide and iron titanate, inorganic brown pigments such as γ-iron oxide, etc., inorganic yellow pigments such as yellow iron oxide, loess, etc., inorganic black pigments such as black iron oxide, carbon black, etc., inorganic violet pigments such as manganese violet, cobalt violet, etc., inorganic green pigments such as chromium hydroxide, chromium oxide, cobalt oxide, cobalt titanate, etc., inorganic blue pigments such as prussian blue, ultramarine blue, etc., those obtained by laking tar pigments, those obtained by laking natural dyes, synthetic resin powders obtained by combining these powders, etc.

Examples of the pearl pigment include titanium oxide-coated mica, titanium oxide-coated mica, bismuth oxychloride, titanium oxide-coated bismuth oxychloride, titanium oxide-coated talc, fish scale foil, titanium oxide-coated colored mica, etc.

Examples of the metal powder pigment include aluminum powder, copper powder, stainless powder, etc.

Examples of the tar pigment include Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 505, Yellow No. 4, Yellow No. 5, Yellow No. 202, Yellow No. 203, Yellow No. 204, Yellow No. 401, Blue No. 1, Blue No. 2, Blue No. 201, Blue No. 404, Green No. 3, Green No. 201, Green No. 204, Green No. 205, Orange No. 201, Orange No. 203, Orange No. 204, Orange No. 206, Orange No. 207, etc.

Examples of the natural pigment include carminic acid, laccaic acid, carthamin, brazilin, crocin, etc.

As these powders, those in which powders are compounded, or those treated with general oil, silicone oil, a fluorine compound, a surfactant, etc. may also be used. One or more of those treated with a hydrolyzable silyl group or an alkyl group having a hydrogen atom directly bonded to a silicon atom, a linear and/or branched organopolysiloxane having a hydrolyzable silyl group or a hydrogen atom directly bonded to a silicon atom, a linear and/or branched organopolysiloxane having a hydrolyzable silyl group or a hydrogen atom directly bonded to a silicon atom and being co-modified by a long chain alkyl, a linear and/or branched organopolysiloxane having a hydrolyzable silyl group or a hydrogen atom directly bonded to a silicon atom and being co-modified by polyoxyalkylene, an acrylic-silicone-based copolymer having a hydrolyzable silyl group or a hydrogen atom directly bonded to a silicon atom, etc., may also be used depending on necessity.

In addition, as the amount of the powder to be contained, a range of 0.1 to 99 mass% based on the entire cosmetic is suitable. Particularly, as the amount to be contained in the case of a powdered solid cosmetic, a range of 80 to 99 mass% based on the entire cosmetic is suitable.

In the inventive cosmetic, one or more surfactants can also be used depending on the purpose. When a surfactant is contained as described, an emulsion can be obtained easily. As such a surfactant, there are anionic, cationic, nonionic and amphoteric active agents, but the surfactant is not particularly limited, and any material can be used as long as it is used in the usual cosmetics.

Examples of the anion surfactant include a fatty acid soap such as sodium stearate and triethanolamine palmitate, an alkyl ether carboxylic acid and a salt thereof, a salt of a condensate of an amino acid and a fatty acid, an alkane sulfonic acid salt, an alkene sulfonic acid salt, a fatty acid ester sulfonic acid salt, a fatty acid amide sulfonic acid salt, a formalin condensation-based sulfonic acid salt, a sulfuric acid ester salt such as an alkyl sulfuric acid ester salt, a secondary higher alcohol sulfuric acid ester salt, an alkyl and allyl ether sulfuric acid ester salt, a sulfuric acid ester salt of a fatty acid ester, a sulfuric acid ester salt of a fatty acid alkylol amide, sulfuric acid ester salts such as turkey red oil, an alkyl phosphoric acid salt, an ether phosphoric acid salt, an alkyl aryl ether phosphoric acid salt, an amide phosphoric acid salt, an N-acyl lactic acid salt, an N-acyl sarcosine salt, and an N-acylamino acid-based active agent.

Examples of the cationic surfactant include an amine salt such as an alkylamine salt, a polyamine and an amino alcohol fatty acid derivative, an alkyl quaternary ammonium salt, an aromatic quaternary ammonium salt, a pyridium salt, and an imidazolium salt.

Examples of the nonionic surfactant include a sorbitan fatty acid ester, a glycerol fatty acid ester, a polyglycerol fatty acid ester, a propylene glycol fatty acid ester, a polyethylene glycol fatty acid ester, a sucrose fatty acid ester, a methyl glucoside fatty acid ester, an alkyl polyglucoside, a polyoxyethylene alkyl ether, a polyoxypropylene alkyl ether, a polyoxyethylene alkyl phenyl ether, a polyoxyethylene fatty acid ester, a polyoxyethylene sorbitan fatty acid ester, a polyoxyethylene sorbitol fatty acid ester, a polyoxyethylene glycerol fatty acid ester, a polyoxyethylene propylene glycol fatty acid ester, a polyoxyethylene castor oil, a polyoxyethylene hardened castor oil, a polyoxyethylene phytostanol ether, a polyoxyethylene phytosterol ether, a polyoxyethylene cholestanol ether, a polyoxyethylene cholesteryl ether, a linear or branched polyoxyalkylene-modified organopolysiloxane, a linear or branched polyoxyalkylene·alkyl-co-modified organopolysiloxane, a linear or branched polyglycerol-modified organopolysiloxane, a linear or branched polyglycerol·alkyl-co-modified organopolysiloxane, an alkanol amide, a sugar ether, and a sugar amide.

Examples of the amphoteric surfactant include betaine, phosphatidylcholine, an aminocarboxylic acid salt, an imidazoline derivative, and an amideamine type. Among these surfactants, a linear or branched organopolysiloxane having a polyoxyalkylene chain or a polyglycerin chain as a hydrophilic group in the molecule or an alkyl-co-modified organopolysiloxane thereof are preferable. Examples of the alkyl-co-modified organopolysiloxane further include a linear or branched organopolysiloxane having a long chain alkyl group having 6 to 20 carbon atoms, in addition to the hydrophilic group.

In addition, in these surfactants, a content of a hydrophilic polyoxyalkylene group or a polyglycerol group preferably occupies 10 to 70 mass% in the molecule, and a contained amount thereof is in the range of 0.1 to 20 mass%, particularly suitable in the range of 0.2 to 10 mass% based on the entire cosmetic.

The inventive cosmetic may contain, as a silicone film former, a silicone resin selected from an acrylic silicone resin and other than the above-described silicone resin (I), a silicone resin (hereinafter, also referred to as silicone resin (II)) composed of components selected from an R₃SiO_{1/2} unit, an R₂SiO_{2/2} unit, an RSiO_{3/2} unit, and an SiO_{4/2} unit (R is the same as the R¹). The acrylic silicone resin is an acrylic-silicone graft copolymer or an acrylic-silicone block copolymer. Furthermore, this acrylic silicone resin is preferably linear. It is also possible to use an acrylic silicone resin containing at least one kind selected from anionic groups such as a pyrrolidinyl group, a long chain alkyl group, a polyoxyalkylene group, and a fluoroalkyl group, and a carboxyl group, etc. in the molecule. When a silicone film former is contained as described, the film-formation ability of the inventive cosmetic can be further enhanced.

The inventive cosmetic can further contain a silicone film former selected from a group consisting of: a silicone resin composed of components selected from R₃SiO_{1/2}, R₂SiO_{2/2}, RSiO_{3/2}, and SiO_{4/2} (R is the same as the R¹) other than the above-described silicone resin; a linear acrylic-silicone graft copolymer; and a linear acrylic-silicone block copolymer.

Examples of the silicone resin (II) include: a resin composed of an R₃SiO_{1/2} unit, an R₂SiO_{2/2} unit, and an SiO_{4/2} unit; a resin composed of an R₃SiO_{1/2} unit and an RSiO_{3/2} unit; a resin composed of an R₃SiO_{1/2} unit, an R₂SiO_{2/2} unit, and an RSiO_{3/2} unit; and a resin composed of an R₃SiO_{1/2} unit, an R₂SiO_{2/2} unit, an RSiO_{3/2} unit, and an SiO_{4/2} unit. It is also possible to use a network silicone containing at least one kind selected from a pyrrolidinyl group, a long chain alkyl group, a polyoxyalkylene group and a fluoroalkyl group, and an amino group in the molecule. When a silicone film former is used, the amount to be contained is preferably 0.1 to 20 mass%, further preferably 1 to 10 mass% based on the total amount of the cosmetic.

In the inventive cosmetic it is also possible to use a composition containing one or more crosslinked organopolysiloxane polymers and an oil agent that is liquid at room temperature depending on the purpose. In this case, the crosslinked organopolysiloxane polymer may have a polyether group and/or a polyglycerin group. In addition, a crosslinked organopolysiloxane polymer containing the above groups and a crosslinked organopolysiloxane polymer not containing the groups may be used in mixture. These crosslinked organopolysiloxane polymers are preferably swollen with respect to the liquid oil agent by containing the liquid oil agent in an amount of its own weight or more. As the liquid oil agent, it is possible to use the liquid silicone oil, hydrocarbon oil, ester oil, natural animal and vegetable oil, semi-synthetic oil, etc., and fluorine-based oil. Examples thereof include lowviscosity silicone oil with 0.65 mm²/sec (25°C) to 100.0 mm²/sec (25°C), hydrocarbon oils such as liquid paraffin, squalane, isododecane, and isohexadecane, glyceride oils such as trioctanoin, ester oils such as isotridecyl isononanoate, N-acyl glutamic acid ester, and lauroyl sarcosinic acid ester, and natural animal and vegetable oils such as macadamia nut oil. In addition, a crosslinking agent of the crosslinked organopolysiloxane polymer is preferably a material having two or more vinylic reactive sites in the molecule, and forming a crosslinked structure by reacting with a hydrogen atom directly bonded to a silicon atom. Examples of the material having two or more vinylic reactive sites in the molecule include an organopolysiloxane having two or more vinyl groups in the molecule, a polyoxyalkylene having two or more allyl groups in the molecule, a polyglycerin having two or more allyl groups in the molecule, α,ω-alkenyldiene, etc. In addition, it is also possible to use a crosslinking agent having at least one selected from the group consisting of a polyoxyalkylene group, a polyglycerin residue, a long chain alkyl group, an alkenyl group, an aryl group, and a fluoroalkyl group. When using the composition containing the crosslinked organopolysiloxane polymer and the oil agent that is liquid at room temperature, the amount to be contained is preferably 0.1 to 80 mass%, further preferably 1 to 50 mass% based on a total amount of the cosmetic.

The inventive cosmetic may contain a siliconemodified olefin wax obtained by subjecting an olefin wax having an unsaturated group obtained by reacting one or more kinds of α-olefins and a diene and an organohydrogen polysiloxane having one or more SiH bonds in one molecule to addition reaction depending on the purpose. As the α-olefin, those having 2 to 12 carbon atoms such as ethylene, propylene, 1-butene, 1-hexene, 4-methyl 1-pentene, etc., are preferable, and as the diene, butadiene, isoprene, 1,4-hexadiene, vinyl norbornene, ethylidene norbornene, dicyclopentadiene, etc., are preferable. As the organohydrogen polysiloxane having a SiH bond, those having a structure of a linear or a siloxane branched type, etc., can be used.

Further, it is possible to add to the inventive cosmetic, a component to be used for usual cosmetics, an oil-soluble gelling agent, an antiperspirant, an ultraviolet light absorbing component (an ultraviolet absorber, an ultraviolet absorptive scattering agent, etc.), a moisturizing agent, an antimicrobial preservative, an antimicrobial agent, a perfume, salts, an antioxidant, a pH adjuster, a chelating agent, a refrigerant, an anti-inflammatory agent, a skin beautifying component (a whitening agent, a cell activator, a rough skin ameliorating agent, a blood circulation promoter, a skin astringent, an antiseborrheic drug, etc.), vitamins, amino acids, a nucleic acid, a hormone, an inclusion compound, a solidifying agent for hair, etc.

Examples of the oil-soluble gelling agent include a gelling agent selected from a metallic soap such as aluminum stearate, magnesium stearate, and zinc myristate; an amino acid derivative such as N-lauroyl-L-glutamic acid and α,γ-di-n-butylamine; a dextrin fatty acid ester such as dextrin palmitic acid ester, dextrin stearic acid ester, and dextrin 2-ethylhexanoic acid / palmitic acid ester; a sucrose fatty acid ester such as sucrose palmitic acid ester and sucrose stearic acid ester; a fructo-oligosaccharide fatty acid ester such as fructo-oligosaccharide stearic acid ester and fructo-oligosaccharide 2-ethylhexanoic acid ester; a benzylidene derivative of sorbitol such as monobenzylidene sorbitol and dibenzylidene sorbitol; and an organically modified clay mineral such as dimethylbenzyldodecylammonium montmorillonite clay and dimethyldioctadecylammonium montmorillonite clay, etc.

Examples of the antiperspirant include an antiperspirant selected from aluminum chlorohydrate, aluminum chloride, aluminum sesquichlorohydrate, zirconyl hydroxychloride, aluminum zirconium hydroxychloride, aluminum zirconium glycine complex, etc.

Examples of the ultraviolet absorber include a benzoic acid-based ultraviolet absorber such as paraaminobenzoic acid, an anthranilic acid-based ultraviolet absorber such as methyl anthranilate, a salicylic acid-based ultraviolet absorber such as methyl salicylate, octyl salicylate, and trimethylcyclohexyl salicylate, a cinnamic acid-based ultraviolet absorber such as octyl para-methoxycinnamate, a benzophenone-based ultraviolet absorber such as 2,4-dihydroxybenzophenone, an urocanic acid-based ultraviolet absorber such as ethyl urocanate, a dibenzoylmethane-based ultraviolet absorber such as 4-t-butyl-4'-methoxy-dibenzoylmethane, phenylbenzimidazole sulfonic acid, a triazine derivative, etc., and examples of the ultraviolet absorptive scattering agent include powder which absorbs or scatters ultraviolet rays such as fine particulate titanium oxide, fine particulate iron-containing titanium oxide, fine particulate zinc oxide, fine particulate cerium oxide and a complex thereof, etc., and a dispersion in which these powders which absorb and scatter ultraviolet rays are dispersed in oil in advance can be also used.

Examples of the moisturizing agent include glycerol, sorbitol, propylene glycol, dipropylene glycol, 1,3-butylene glycol, pentylene glycol, glucose, xylitol, maltitol, polyethylene glycol, hyaluronic acid, chondroitin sulfate, pyrrolidone carboxylate, polyoxyethylene methyl glucoside, polyoxypropylene methyl glucoside, egg yolk lecithin, soybean lecithin, phosphatidyl choline, phosphatidyl ethanolamine, phosphadithyl serine, phosphatidyl glycerol, phosphatidylinositol, and sphingo-phospholipid.

Examples of the antimicrobial preservative include paraoxybenzoic acid alkyl ester, benzoic acid, sodium benzoate, sorbic acid, potassium sorbate, and phenoxyethanol; and examples of the antimicrobial agent include benzoic acid, salicylic acid, carbolic acid, sorbic acid, paraoxybenzoic acid alkyl ester, parachlorometacresol, hexachlorophene, benzalkonium chloride, chlorhexidine chloride, trichlorocarbanilide, a photosensitizer, and phenoxyethanol.

Examples of the perfume include natural perfumes and synthetic perfumes. Examples of the natural perfume include vegetable perfume separated from flowers, leaves, wood, and pericarp; and animal perfume such as musk and civet. Examples of the synthetic perfume include hydrocarbons such as monoterpene; alcohols such as an aliphatic alcohol and an aromatic alcohol; aldehydes such as terpene aldehyde and aromatic aldehyde; ketones such as an alicyclic ketone; esters such as a terpenebased ester; lactones; phenols; oxides; nitrogencontaining compounds; acetals; etc.

Examples of the salts include an inorganic salt, an organic salt, an amine salt and an amino acid salt. Examples of the inorganic salt include a sodium salt, a potassium salt, a magnesium salt, a calcium salt, an aluminum salt, a zirconium salt, a zinc salt of an inorganic acid such as hydrochloric acid, sulfuric acid, carbonic acid, and nitric acid; examples of the organic salt include a salt of an organic acid such as acetic acid, dehydroacetic acid, citric acid, malic acid, succinic acid, ascorbic acid, and stearic acid; and examples of the amine salt and the amino acid salt include a salt of an amine such as triethanolamine and a salt of an amino acid such as glutamic acid. In addition, as others, salts of hyaluronic acid and chondroitin sulfuric acid, etc., aluminum zirconium glycine complex, etc., and further an acid-alkali neutralizing salt used in cosmetic formulation, etc., can also be used.

Examples of the antioxidant include tocopherol, p-t-butylphenol, butylhydroxyanisole, dibutylhydroxytoluene, and phytic acid; examples of the pH adjuster include lactic acid, citric acid, glycolic acid, succinic acid, tartaric acid, dl-malic acid, potassium carbonate, sodium hydrogen carbonate, and ammonium hydrogen carbonate; examples of the chelating agent include alanine, sodium edetate, sodium polyphosphate, sodium metaphosphate, and phosphoric acid; examples of the refrigerant include L-menthol and camphor; and examples of the anti-inflammatory agent include allantoin, glycyrrhizic acid and a salt thereof, glycyrrhetinic acid and stearyl glycyrrhetinate, tranexamic acid, and azulene.

Examples of the skin beautifying component include a whitening agent such as a placenta extract, arbutin, glutathione, and a saxifrage extract; a cell activator or a rough skin ameliorating agent such as a royal jelly, a photosensitizer, a cholesterol derivative, and a bovine blood extract; a blood circulation promoter such as nonylic acid vanillylamide, benzyl nicotinate, β-butoxyethyl nicotinate, capsaicin, zingerone, cantharides tincture, ichthammol, caffeine, tannic acid, α-borneol, tocopherol nicotinate, inositol hexanicotinate, cyclandelate, cinnarizine, tolazoline, acetylcholine, verapamil, cepharanthin, and γ-oryzanol; a skin astringent such as zinc oxide and tannic acid; and an antiseborrheic drug such as sulfur and thiantrol.

Examples of the vitamins include vitamin A such as vitamin A oil, retinol, retinol acetate, and retinol palmitate; vitamin B2 such as riboflavin, riboflavin butyrate, and flavin adenine nucleotide; vitamin B6 such as pyridoxine hydrochloride, pyridoxine dioctanoate, and pyridoxine tripalmitate; vitamin B such as vitamin B12 and a derivative thereof, and vitamin B15 and a derivative thereof; vitamin C such as L-ascorbic acid, L-ascorbic acid dipalmitate, L-ascorbic acid-2-sulfate sodium, and L-ascorbic acid phosphate diester dipotassium; vitamin D such as ergocalciferol and cholecalciferol; vitamin E such as α-tocopherol, β-tocopherol, γ-tocopherol, dl-α-tocopherol acetate, dl-α-tocopherol nicotinate, and dl-α-tocopherol succinate; vitamin H; vitamin P; nicotinic acids such as nicotinic acid, benzyl nicotinate, and nicotinic acid amide; pantothenic acids such as calcium pantothenate, D-pantothenyl alcohol, pantothenyl ethyl ether, and acetyl pantothenyl ethyl ether; and biotin.

Examples of the amino acids include glycine, valine, leucine, isoleucine, serine, threonine, phenylalanine, arginine, lysine, aspartic acid, glutamic acid, cystine, cysteine, methionine, and tryptophan; examples of the nucleic acid include deoxyribonucleic acid; and examples of the hormone include estradiol and ethenyl estradiol.

Example of the inclusion compound includes cyclodextrin.

Examples of the solidifying agent for hair include amphoteric, anionic, cationic, and nonionic polymer compounds, and include a polyvinylpyrrolidone-based polymer compound such as polyvinyl-pyrrolidone and a vinylpyrrolidone/vinyl acetate copolymer; an acidic vinyl ether-based polymer compound such as a methyl vinyl ether/maleic anhydride alkyl half ester copolymer; an acidic polyvinyl acetate-based polymer such as a vinyl acetate/crotonic acid copolymer; an acidic acrylic-based polymer compound such as a (meth)acrylic acid/alkyl (meth)acrylate copolymer and a (meth)acrylic acid/alkyl (meth)acrylate/alkyl acrylamide copolymer; and an amphoteric acrylic-based polymer compound such as an N-methacryloylethyl-N,N-dimethyl-ammonium·α-N-methylcarboxybetaine/alkyl (meth)acrylate copolymer and a hydroxypropyl (meth)acrylate/butylaminoethyl methacrylate/octylamide acrylate copolymer. In addition, a naturally-derived polymer compound such as cellulose or a derivative thereof, and keratin and collagen or a derivative thereof can also be suitably used.

In the present invention, dosage form or form of the cosmetic is not particularly limited, and may be aqueous, oily, emulsion-based (water-in-oil type emulsion, oil-in water type emulsion, non-aqueous emulsion, multi-emulsion such as W/O/W and O/W/O, etc.), suspension, paste, or solid.

The use of the cosmetic may also be arbitrary. Examples include skin care cosmetics such as skin lotion, milky lotion, cream, cleansing, pack, oil liquid, massaging agent, beauty essence, beauty oil, hand cream, lip cream, and wrinkle concealment; make-up cosmetics such as make-up base, concealer, white powder, powder foundation, liquid foundation, cream foundation, oil foundation, blusher, eye shadow, mascara, eyeliner, eyebrow, and lipstick; hair cosmetics such as shampoo, rinse, treatment, and setting agent; ultraviolet protective cosmetics such as sunscreen oil and sunscreen milky lotion, and sunscreen cream; and other detergents, deodorants, antiperspirants, etc.

In the present invention, the cosmetic is preferably one of a water-based, oil-based, or emulsion-based skin care cosmetic, hair cosmetic, antiperspirant, make-up cosmetic, or ultraviolet protection cosmetic.

### EXAMPLE

Hereinafter, the present invention will be described more specifically with reference to Examples and Comparative Examples, but the present invention is not limited to the following Examples. The molar ratio of an R₃SiO_{1/2} unit, an R₂SiO_{2/2} unit, an RSiO_{3/2} unit, and an SiO_{4/2} unit was calculated by Si-NMR measurement. The weight-average molecular weight was determined in terms of polystyrene in gel permeation chromatography (GPC) analysis.

### [Synthesis Example 1]

As raw materials, 150 g of hexamethyldisiloxane, 433 g of triethoxymethylsilane, and 219 g of ethyl polysilicate containing 40% of an SiO₂ component, and as a solvent, 300 g of IPA (isopropyl alcohol) were charged into a reactor. 6 g of methanesulfonic acid was added and the mixture was cooled to 10 to 20°C, and 180 g of water was dropped thereto while stirring. After the dropping was completed, the mixture was heated at 70 to 90°C for 5 hours to perform hydrolysis condensation, and thus, a silicone resin solution was obtained. Next, 9.6 g of a 25% aqueous sodium hydroxide solution and 1.24 g of calcium carbonate were added thereto to neutralize the acid. After that, 400 g of decamethylcyclopentasiloxane was added thereto, and the mixture was heated to 130°C to remove the generated ethanol and excess water and then cooled. Furthermore, after distillation under reduced pressure, the solution was diluted with decamethylcyclopentasiloxane to adjust the silicone resin concentration to 50%, and then filtered. Thus, a 50% solution of silicone resin in decamethylcyclopentasiloxane was obtained, the silicone resin having a weight-average molecular weight of 26000, and the molar ratio of (CH₃)₃SiO_{1/2} unit:CH₃SiO_{3/2} unit:SiO_{4/2} unit being 0.32:0.42:0.26.

### [Synthesis Example 2]

Except that the raw materials were changed to 135 g of hexamethyldisiloxane, 174 g of triethoxymethylsilane, and 293 g of ethyl polysilicate containing 40% of an SiO₂ component, hydrolysis condensation and neutralization were carried out in the same manner as in Synthesis Example 1. After that, 400 g of tristrimethylsiloxy methylsilane was added thereto, and the mixture was heated to 130°C to remove the generated ethanol and excess water and then cooled. Furthermore, after distillation under reduced pressure, the solution was diluted with tristrimethylsiloxy methylsilane to adjust the silicone resin concentration to 50%, and then filtered. Thus, a 50% solution of silicone resin in tristrimethylsiloxy methylsilane was obtained, the silicone resin having a weight-average molecular weight of 21000, and the molar ratio of (CH₃)₃SiO_{1/2} unit: CH₃SiO_{3/2} unit:SiO_{4/2} unit being 0.36:0.21:0.43.

### [Synthesis Example 3]

Except that the raw materials were changed to 100 g of hexamethyldisiloxane, 282 g of triethoxymethylsilane, and 158 g of ethyl polysilicate containing 40% of an SiO₂ component, hydrolysis condensation and neutralization were carried out in the same manner as in Synthesis Example 1. After that, 450 g of isododecane was added thereto, and the mixture was heated to 130°C to remove the generated ethanol and excess water and then cooled. Furthermore, after distillation under reduced pressure, the solution was diluted with isododecane to adjust the silicone resin concentration to 50%, and then filtered. Thus, a 50% solution of silicone resin in isododecane was obtained, the silicone resin having a weight-average molecular weight of 25000, and the molar ratio of (CH₃) ₃SiO_{1/2} unit:CH₃SiO_{3/2} unit:SiO_{4/2} unit being 0.32:0.41:0.27.

### [Synthesis Example 4]

Except that the raw materials were changed to 200 g of hexamethyldisiloxane, 878 g of triethoxymethylsilane, and 296 g of ethyl polysilicate containing 40% of an SiO₂ component, hydrolysis condensation and neutralization were carried out in the same manner as in Synthesis Example 1. After that, 450 g of isododecane was added thereto, and the mixture was heated to 130°C to remove the generated ethanol and excess water and then cooled. Furthermore, after distillation under reduced pressure, the solution was diluted with isododecane to adjust the silicone resin concentration to 50%, and then filtered. Thus, a 50% solution of silicone resin in isododecane was obtained, the silicone resin having a weight-average molecular weight of 46000, and the molar ratio of (CH₃) ₃SiO_{1/2} unit:CH₃SiO_{3/2} unit:SiO_{4/2} unit being 0.26:0.53:0.21.

### [Synthesis Example 5]

Except that the raw materials were changed to 150 g of hexamethyldisiloxane, 63 g of diethoxydimethylsilane, 304 g of triethoxymethylsilane, and 256 g of ethyl polysilicate containing 40% of an SiO₂ component, hydrolysis condensation and neutralization were carried out in the same manner as in Synthesis Example 1. After that, 420 g of isododecane was added thereto, and the mixture was heated to 130°C to remove the generated ethanol and excess water and then cooled. Furthermore, after distillation under reduced pressure, the solution was diluted with isododecane to adjust the silicone resin concentration to 50%, and then filtered. Thus, a 50% solution of silicone resin in isododecane was obtained, the silicone resin having a weight-average molecular weight of 29500, and the molar ratio of (CH₃)₃SiO_{1/2} unit: (CH₃)₂SiO_{2/2} unit:CH₃SiO_{3/2} unit:SiO_{4/2} unit being 0.33:0.07:0.30:0.30.

### [Synthesis Example 6]

Except that the raw materials were changed to 100 g of hexamethyldisiloxane, 234 g of triethoxymethylsilane, and 172 g of ethyl polysilicate containing 40% of an SiO₂ component, hydrolysis condensation and neutralization were carried out in the same manner as in Synthesis Example 1. After that, 350 g of isododecane was added thereto, and the mixture was heated to 130°C to remove the generated ethanol and excess water and then cooled. Furthermore, after distillation under reduced pressure, the solution was diluted with isododecane to adjust the silicone resin concentration to 50%, and then filtered. Thus, a 50% solution of silicone resin in isododecane was obtained, the silicone resin having a weight-average molecular weight of 31000, and the molar ratio of (CH₃)₃SiO_{1/2} unit:CH₃SiO_{3/2} unit:SiO_{4/2} unit being 0.33:0.36:0.31.

### [Synthesis Example 7]

Except that the raw materials were changed to 100 g of hexamethyldisiloxane, 24 g of diethoxydimethylsilane, 117 g of triethoxymethylsilane, and 148 g of ethyl polysilicate containing 40% of an SiO₂ component, hydrolysis condensation and neutralization were carried out in the same manner as in Synthesis Example 1. After that, 430 g of decamethylcyclopentasiloxane was added thereto, and the mixture was heated to 130°C to remove the generated ethanol and excess water and then cooled. Furthermore, after distillation under reduced pressure, the solution was diluted with decamethylcyclopentasiloxane to adjust the silicone resin concentration to 50%, and then filtered. Thus, a 50% solution of silicone resin in decamethylcyclopentasiloxane was obtained, the silicone resin having a weight-average molecular weight of 21000, and the molar ratio of (CH₃) ₃SiO_{1/2} unit:CH₃SiO_{3/2} unit:SiO_{4/2} unit being 0.41:0.05:0.22:0.32.

### [Synthesis Example 8]

Except that the raw materials were changed to 225 g of hexamethyldisiloxane, 34 g of diethoxydimethylsilane, 412 g of triethoxymethylsilane, and 450 g of ethyl polysilicate containing 40% of an SiO₂ component, hydrolysis condensation and neutralization were carried out in the same manner as in Synthesis Example 1. After that, 450 g of isododecane was added thereto, and the mixture was heated to 130°C to remove the generated ethanol and excess water and then cooled. Furthermore, after distillation under reduced pressure, the solution was diluted with isododecane to adjust the silicone resin concentration to 50%, and then filtered. Thus, a 50% solution of silicone resin in isododecane was obtained, the silicone resin having a weight-average molecular weight of 30500, and the molar ratio of (CH₃) ₃SiO_{1/2} unit: (CH₃) ₂SiO_{2/2} unit:CH₃SiO_{3/2} unit:SiO_{4/2} unit being 0.33:0.03:0.28:0.36.

### [Synthesis Example 9]

Except that the raw materials were changed to 120 g of hexamethyldisiloxane, 231 g of triethoxymethylsilane, and 222 g of ethyl polysilicate containing 40% of an SiO₂ component, hydrolysis condensation and neutralization were carried out in the same manner as in Synthesis Example 1. After that, 350 g of isododecane was added thereto, and the mixture was heated to 130°C to remove the generated ethanol and excess water and then cooled. Furthermore, after distillation under reduced pressure, the solution was diluted with isododecane to adjust the silicone resin concentration to 50%, and then filtered. Thus, a 50% solution of silicone resin in isododecane was obtained, the silicone resin having a weight-average molecular weight of 28000, and the molar ratio of (CH₃) ₃SiO_{1/2} unit:CH₃SiO_{3/2} unit:SiO_{4/2} unit being 0.35:0.30:0.35.

### [Synthesis Example 10]

Except that the raw materials were changed to 100 g of hexamethyldisiloxane, 21 g of diethyldiethoxysilane, 258 g of triethoxymethylsilane, and 163 g of ethyl polysilicate containing 40% of an SiO₂ component, hydrolysis condensation and neutralization were carried out in the same manner as in Synthesis Example 1. After that, 360 g of decamethylcyclopentasiloxane was added thereto, and the mixture was heated to 130°C to remove the generated ethanol and excess water and then cooled. Furthermore, after distillation under reduced pressure, the solution was diluted with decamethylcyclopentasiloxane to adjust the silicone resin concentration to 50%, and then filtered. Thus, a 50% solution of silicone resin in decamethylcyclopentasiloxane was obtained, the silicone resin having a weight-average molecular weight of 28500, and the molar ratio of (CH₃) ₃SiO_{1/2} unit: (CH₃)₂SiO_{2/2} unit:CH₃SiO_{3/2} unit:SiO_{4/2} unit being 0.31:0.04:0.37:0.28.

### [Comparative Synthesis Example 1]

As raw materials, 150 g of hexamethyldisiloxane, 415 g of triethoxymethylsilane, and 245 g of ethyl polysilicate containing 40% of an SiO₂ component, and as a solvent, 300 g of IPA were charged into a reactor. 6 g of methanesulfonic acid was added and the mixture was cooled to 10 to 20°C, and 180 g of water was dropped thereto while stirring. After the dropping was completed, the mixture was heated at 70 to 90°C for 5 hours to perform hydrolysis condensation, and thus, a silicone resin solution was obtained. Next, 9.6 g of a 25% aqueous sodium hydroxide solution and 1.24 g of calcium carbonate were added thereto to neutralize the acid. After that, 400 g of decamethylcyclopentasiloxane was added thereto, and the mixture was heated to 130°C to remove the generated ethanol and excess water and then cooled. Furthermore, after distillation under reduced pressure, the solution was diluted with decamethylcyclopentasiloxane, and then filtered. Thus, a 50% solution of silicone resin in decamethylcyclopentasiloxane was obtained, the silicone resin having a weight-average molecular weight of 9350, and the molar ratio of (CH₃)₃SiO_{1/2} unit:CH₃SiO_{3/2} unit:SiO_{4/2} unit being 0.32:0.40:0.28.

### [Comparative Synthesis Example 2]

Except that the raw materials were changed to 100 g of hexamethyldisiloxane, 308 g of triethoxymethylsilane, and 297 g of ethyl polysilicate containing 40% of an SiO₂ component, the same operation as Comparative Synthesis Example 1 was carried out. Thus, a 50% solution of silicone resin in decamethylcyclopentasiloxane was obtained, the silicone resin having a weight-average molecular weight of 4550, and the molar ratio of (CH₃)₃SiO_{1/2} unit:CH₃SiO_{3/2} unit:SiO_{4/2} unit being 0.25:0.35:0.40.

### [Comparative Synthesis Example 3]

Except that the raw materials were changed to 120 g of hexamethyldisiloxane, 527 g of triethoxymethylsilane, and 136 g of ethyl polysilicate containing 40% of an SiO₂ component, the same operation as Comparative Synthesis Example 1 was carried out. Thus, a 50% solution of silicone resin in decamethylcyclopentasiloxane was obtained, the silicone resin having a weight-average molecular weight of 25500, and the molar ratio of (CH₃)₃SiO_{1/2} unit:CH₃SiO_{3/2} unit:SiO_{4/2} unit being 0.28:0.55:0.17.

### [Comparative Synthesis Example 4]

Except that the raw materials were changed to 150 g of hexamethyldisiloxane, 154 g of triethoxymethylsilane, and 480 g of ethyl polysilicate containing 40% of an SiO₂ component, the 600 g of decamethylcyclopentasiloxane was changed to 300 g of isododecane, and that the diluent used after the distillation under reduced pressure was changed to isododecane, the same operation as Comparative Synthesis Example 1 was carried out. Thus, a 50% solution of silicone resin in isododecane was obtained, the silicone resin having a weight-average molecular weight of 24000, and the molar ratio of (CH₃) ₃SiO_{1/2} unit:CH₃SiO_{3/2} unit:SiO_{4/2} unit being 0.31:0.15:0.54.

### [Comparative Synthesis Example 5]

Except that the raw materials were changed to 120 g of hexamethyldisiloxane and 791 g of triethoxymethylsilane, the same operation as Comparative Synthesis Example 1 was carried out. Thus, a 50% solution of silicone resin in decamethylcyclopentasiloxane was obtained, the silicone resin having a weight-average molecular weight of 3190, and the molar ratio of (CH₃)₃SiO_{1/2} unit:CH₃SiO_{3/2} unit being 0.25:0.75.

### [Comparative Synthesis Example 6]

Except that the raw materials were changed to 150 g of hexamethyldisiloxane and 420 g of ethyl polysilicate containing 40% of an SiO₂ component, the 400 g of decamethylcyclopentasiloxane was changed to 300 g of isododecane, and that the diluent used after the distillation under reduced pressure was changed to isododecane, the same operation as Comparative Synthesis Example 1 was carried out. Thus, a 50% solution of silicone resin in isododecane was obtained, the silicone resin having a weight-average molecular weight of 5220, and the molar ratio of (CH₃)₃SiO_{1/2} unit:SiO_{4/2} unit being 0.40:0.60.

### [Comparative Synthesis Example 7]

Except that the raw materials were changed to 120 g of hexamethyldisiloxane, 264 g of triethoxymethylsilane, and 148 g of ethyl polysilicate containing 40% of an SiO₂ component, the same operation as Comparative Synthesis Example 1 was carried out. Thus, a 50% solution of silicone resin in decamethylcyclopentasiloxane was obtained, the silicone resin having a weight-average molecular weight of 19500, and the molar ratio of (CH₃)₃SiO_{1/2} unit: CH₃SiO_{3/2} unit:SiO_{4/2} unit being 0.38:0.38:0.24.

The weight-average molecular weight and units of each silicone resin obtained in Synthesis Examples 1 to 10 and Comparative Synthesis Examples 1 to 7 were as shown in the Table 1 below. In addition, the R₃SiO_{1/2} unit, the R₂SiO_{2/2} unit, the RSiO_{3/2} unit, and the SiO_{4/2} unit were respectively denoted as described below. R₃SiO_{1/2} unit→M, R₂SiO_{2/2} unit→D, RSiO_{3/2} unit→T, and SiO_{4/2} unit→Q

Note that each of the M/D/T/Q in the Table respectively corresponds to the value of "a", "b", "c" , and "d" of the general formulae (A) to (D), M/(T+Q) corresponds to a/(c+d), and T/Q corresponds to c/d.

**[Table 1]**

| | Weight-average molecular weight | M/D/T/Q | M/ (T+Q) | T/Q |
|---|---|---|---|---|
| Synthesis Example 1 | 26000 | 0.32/0/0.42/0.26 | 0.47 | 1. 62 |
| Synthesis Example 2 | 21000 | 0.36/0/0.21/0.43 | 0.56 | 0.49 |
| Synthesis Example 3 | 25000 | 0.32/0/0.41/0.27 | 0.47 | 1.52 |
| Synthesis Example 4 | 46000 | 0.26/0/0.53/0.21 | 0.35 | 2.52 |
| Synthesis Example 5 | 29500 | 0.33/0.07/0.3/0.3 | 0.55 | 1.00 |
| Synthesis Example 6 | 31000 | 0.33/0/0.36/0.31 | 0.49 | 1.09 |
| Synthesis Example 7 | 21000 | 0.41/0.05/0.22/0.32 | 0.76 | 0.69 |
| Synthesis Example 8 | 30500 | 0.33/0.03/0.28/0.36 | 0.52 | 0.78 |
| Synthesis Example 9 | 28000 | 0.35/0/0.3/0.35 | 0.54 | 0.86 |
| Synthesis Example 10 | 28500 | 0.31/0.04/0.37/0.28 | 0.48 | 1.32 |
| Comparative Synthesis Example 1 | 9350 | 0.32/0/0.4/0.28 | 0.47 | 1. 43 |
| Comparative Synthesis Example 2 | 4550 | 0.25/0/0.35/0.4 | 0.33 | 0.88 |
| Comparative Synthesis Example 3 | 25500 | 0.28/0/0.55/0.17 | 0.39 | 3.24 |
| Comparative Synthesis Example 4 | 24000 | 0.31/0/0.15/0.54 | 0.45 | 0.28 |
| Comparative Synthesis Example 5 | 3190 | 0.25/0/0.75/0 | 0.33 | - |
| Comparative Synthesis Example 6 | 5220 | 0.4/0/0/0.6 | 0.67 | 0.0 |
| Comparative Synthesis Example 7 | 19500 | 0.38/0/0.38/0.24 | 0.60 | 1.50 |

The solvent of the solution of silicone resin obtained in the Synthesis Examples and Comparative Synthesis Examples were distilled off, and a film was formed. Then, appearance, stickiness, and brittleness due to friction were evaluated. Table 2 shows the results.

**[Table 2]**

| | Appearance of film | Stickiness | Brittleness |
|---|---|---|---|
| Synthesis Example 1 | Cracked film | Absent | Present |
| Synthesis Example 2 | Cracked film | Absent | Present |
| Synthesis Example 3 | Cracked film | Absent | Present |
| Synthesis Example 4 | Cracked film | Absent | Present |
| Synthesis Example 5 | Cracked film | Absent | Present |
| Synthesis Example 6 | Cracked film | Absent | Present |
| Synthesis Example 7 | Cracked film | Absent | Present |
| Synthesis Example 8 | Cracked film | Absent | Present |
| Synthesis Example 9 | Cracked film | Absent | Present |
| Synthesis Example 10 | Cracked film | Absent | Present |
| Comparative Synthesis Example 1 | Uniform continuous film | Absent | Absent |
| Comparative Synthesis Example 2 | Uniform continuous film | Absent | Absent |
| Comparative Synthesis Example 3 | Uniform continuous film | Present | Absent |
| Comparative Synthesis Example 4 | Cracked film | Absent | Present |
| Comparative Synthesis Example 5 | Highly viscous liquid | Present | - |
| Comparative Synthesis Example 6 | Cracked film | Absent | Present |
| Comparative Synthesis Example 7 | Cracked film | Absent | Present |

In each of Synthesis Examples 1 to 10, a cracked film was obtained, and there was no stickiness. In each of Comparative Synthesis Examples 1 and 2, the molecular weight was low, and a uniform continuous film having no stickiness was obtained. In Comparative Synthesis Example 3, a sticky uniform continuous film was obtained since many T units were contained. In Comparative Synthesis Example 4, many Q units were contained, but as in Synthesis Examples 1 to 10, a cracked film was obtained, and there was no stickiness. In Comparative Synthesis Example 5, a film was not formed, and a highly viscous liquid was obtained since no Q units were contained, and the silicone resin was composed of M units and T units. In Comparative Synthesis Example 6, as in Synthesis Examples 1 to 10, a cracked film was obtained, and there was no stickiness since no T units were contained, and the silicone resin was composed of M units and Q units. In Comparative Synthesis Example 7, as in Synthesis Examples 1 to 10, a cracked film was obtained, and there was no stickiness, but the weight-average molecular weight was outside the range of the present invention.

The silicone resin to be contained in the inventive cosmetic gives a hard, brittle film on its own as described above. However, as described below, a cosmetic containing this silicone resin can be a cosmetic that has no stickiness upon application, has smooth spreadability, excellent feeling on use, good water resistance, and excellent cosmetic durability owing to good adhesion to skin, can prevent adhesion to clothes or the like (secondary adhesion), and has excellent abrasion resistance.

### <Examples 1 to 3 and Comparative Examples 1 to 4>

Emulsion cream foundations of the compositions shown in Table 3 were prepared.

**[Table 3]**

| Numbers | Composition (mass%) | Example | | | Comparative Example | | | |
|---|---|---|---|---|---|---|---|---|
| | | 1 | 2 | 3 | 1 | 2 | 3 | 4 |
| 1 | Crosslinked polyether-modified silicone (Note 1) | 5.0 | ← | ← | ← | ← | ← | ← |
| 2 | Crosslinked dimethylpolysiloxane (Note 2) | 6.0 | ← | ← | ← | ← | ← | - |
| 3 | Polyether-modified silicone (Note 3) | 1.0 | ← | ← | ← | ← | ← | ← |
| 4 | Dimethylpolysiloxane (Note 4) | 2.0 | ← | ← | ← | ← | ← | ← |
| 5 | Decamethylcyclopentasiloxane | 6.3 | ← | ← | ← | ← | ← | ← |
| 6 | Triethylhexanoin | 4.0 | ← | ← | ← | ← | ← | ← |
| 7 | Neopentyl glycol dioctanoate | 2.0 | ← | ← | ← | ← | ← | ← |
| 8 | Polymethylsilsesquioxane powder (Note 5) | 1.5 | ← | ← | ← | ← | ← | ← |
| 9 | 1,3-BG | 5.0 | ← | ← | ← | ← | ← | ← |
| 10 | Sodium chloride | 0.5 | ← | ← | ← | ← | ← | ← |
| 11 | Water | 50.0 | ← | ← | ← | ← | ← | ← |
| 12 | Silicone-treated titanium oxide (Note 6) | 8.65 | ← | ← | ← | ← | ← | ← |
| 13 | Silicone-treated red iron oxide (Note 6) | 0.45 | ← | ← | ← | ← | ← | ← |
| 14 | Silicone-treated yellow iron oxide (Note 6) | 0.75 | ← | ← | ← | ← | ← | ← |
| 15 | Silicone-treated black iron oxide (Note 6) | 0.15 | ← | ← | ← | ← | ← | ← |
| 16 | Dissolved product of Synthesis Example 1 | 5.0 | - | - | - | - | - | - |
| 17 | Dissolved product of Synthesis Example 6 | - | 5.0 | - | - | - | - | - |
| 18 | Dissolved product of Synthesis Example 9 | - | - | 5.0 | - | - | - | - |
| 19 | Dissolved product of Comparative Synthesis Example 1 | - | - | - | 5.0 | - | - | - |
| 20 | Dissolved product of Comparative Synthesis Example 4 | - | - | - | - | 5.0 | - | - |
| 21 | Dissolved product of Comparative Synthesis Example 6 | - | - | - | - | - | 5.0 | - |
| 22 | Dissolved product of Comparative Synthesis Example 7 | - | - | - | - | - | - | 5.0 |
| 23 | Antioxidant | 0.5 | ← | ← | ← | ← | ← | ← |
| 24 | Antiseptic | 1.0 | ← | ← | ← | ← | ← | ← |
| 25 | Perfume | 0.2 | ← | ← | ← | ← | ← | ← |
| Total | | 100 | ← | ← | ← | ← | ← | ← |

| | | | | | | | | |
|---|---|---|---|---|---|---|---|---|
| (Note 1) KSG-210: manufactured by Shin-Etsu Chemical Co., Ltd. (Note 2) KSG-15: manufactured by Shin-Etsu Chemical Co., Ltd. (Note 3) KF-6017: manufactured by Shin-Etsu Chemical Co., Ltd. (Note 4) KF-96A-6cs: manufactured by Shin-Etsu Chemical Co., Ltd. (Note 5) KMP-590: manufactured by Shin-Etsu Chemical Co., Ltd. (Note 6) KF-9909: manufactured by Shin-Etsu Chemical Co., Ltd., treatment | | | | | | | | |

The "←" in Table 3 means the contained amount was the same as in Example 1.

### [Preparation of cosmetics]

The components (1) to (4), part of the component (5), the components (6) to (8), (16) to (23), and (24) were stirred and mixed to be homogeneous. The components (9) and (10) dissolved separately and uniformly in the component (11) were gently added to the mixture and emulsified. The components (12) to (15), the remainder of the component (5), and the component (25) were added to the emulsion and mixed. The mixture was loaded into a prescribed container to prepare an emulsion cream foundation. The following evaluations were conducted on the resulting emulsion cream foundations.

### [Usability evaluation]

Spreading at the time of application (spreadability), sticky feeling, finished color unevenness, cosmetic durability (durability (evaluation 8 hours after application)), and secondary adhesion of the resulting emulsion cream foundations were evaluated by 50 female expert panelists according to the following criteria and the evaluation results were averaged.

### [Secondary adhesion prevention effect]

The emulsion cream foundations were applied to the foreheads of the expert panelists respectively by the same operation, and at 20 minutes after the application, tissue paper was pressed against the applied part, and the secondary adhesion prevention effect of the cosmetic was evaluated according to the criteria of the following Table 4. The average of the evaluation results was taken.

**[Table 4]**

| Points | Spreadability | Sticky feeling | Color unevenness | Cosmetic durability | Secondary adhesion |
|---|---|---|---|---|---|
| 5 | Good | None | None | Good | None |
| 4 | Slightly good | Nearly none | Nearly none | Slightly good | Nearly none |
| 3 | Normal | Normal | Normal | Normal | Normal |
| 2 | Slightly bad | Slightly sticky | Slightly uneven | Slightly bad | Slight |
| 1 | Bad | Sticky | Considerably uneven | Bad | Considerable adhesion |

On the basis of the average points of the obtained evaluation results, the results are shown in Table 5 based on the following.
Excellent: the average point is 4.5 points or more
Good: the average point is 4.0 points or more and less than 4.5 points
Fair: the average point is 3.0 points or more and less than 4.0 points
Poor: the average point is 2.0 points or more and less than 3.0 points
Bad: the average point is less than 2.0 points

**[Table 5]**

| Items | Example 1 | Example 2 | Example 3 | Comparative Example 1 | Comparative Example 2 | Comparative Example 3 | Comparative Example 4 |
|---|---|---|---|---|---|---|---|
| Spreadability | Good | Good | Good | Good | Fair | Poor | Good |
| Sticky feeling | Excellent | Excellent | Excellent | Good | Excellent | Fair | Good |
| Color unevenness | Good | Excellent | Good | Good | Poor | Bad | Good |
| Cosmetic durability | Excellent | Excellent | Excellent | Good | Excellent | Fair | Good |
| Secondary adhesion | Excellent | Excellent | Excellent | Good | Poor | Bad | Good |

As evident from Table 5, the inventive cosmetics (Examples 1 to 3) were significantly superior to Comparative Example 1 and Comparative Example 4 regarding sticky feeling, cosmetic durability, and secondary adhesion prevention, since the molecular weight of the film was high. In addition, the inventive cosmetics were significantly superior to Comparative Example 2 regarding spreadability, color unevenness, and secondary adhesion prevention, since the amount of T units contained in the film was large. Furthermore, the inventive cosmetics were significantly superior to Comparative Example 3 regarding spreadability, sticky feeling, color unevenness, cosmetic durability, and secondary adhesion prevention, since the molecular weight of the film was high and T units were contained.

### [Examples 4, 5, and Comparative Examples 5, 6, and 7]

Lipsticks of the compositions shown in Table 6 were prepared.

**[Table 6]**

| Numbers | Composition (mass%) | Example | | Comparative Example | | |
|---|---|---|---|---|---|---|
| | | 4 | 5 | 5 | 6 | 7 |
| 1 | Candelilla wax | 4.0 | ← | ← | ← | ← |
| 2 | Polyethylene | 2.0 | ← | ← | ← | ← |
| 3 | Microcrystalline wax | 3.0 | ← | ← | ← | ← |
| 4 | Ceresin | 7.0 | ← | ← | ← | ← |
| 5 | Stearyl-modified acrylic silicone resin (Note 1) | 14.0 | ← | ← | ← | ← |
| 6 | Diphenyl dimethicone (Note 2) | 17.8 | ← | ← | ← | ← |
| 7 | Dissolved product of Synthesis Example 1 | 6.0 | - | - | - | - |
| 8 | Dissolved product of Synthesis Example 6 | - | 6.0 | - | - | - |
| 9 | Dissolved product of Comparative Synthesis Example 1 | - | - | 6.0 | - | - |
| 10 | Dissolved product of Comparative Synthesis Example 4 | - | - | - | 6.0 | - |
| 11 | Dissolved product of Comparative Synthesis Example 7 | - | - | - | - | 6.0 |
| 12 | Alkyl-modified branched polyglycerol-modified silicone (Note 3) | 3.0 | ← | ← | ← | ← |
| 13 | Macadamia nut oil | 15.0 | ← | ← | ← | ← |
| 14 | Hydrogenated polyisobutene | 8.0 | ← | ← | ← | ← |
| 15 | Isotridecyl isononanoate | 5.0 | ← | ← | ← | ← |
| 16 | Perfume | 0.2 | ← | ← | ← | ← |
| 17 | Lipstick pigment | 10 | ← | ← | ← | ← |
| 18 | Mica | 5 | ← | ← | ← | ← |
| Total | | 100 | ← | ← | ← | ← |

| | | | | | | |
|---|---|---|---|---|---|---|
| (Note 1) KP-561P: manufactured by Shin-Etsu Chemical Co., Ltd. (Note 2) KF-54: manufactured by Shin-Etsu Chemical Co., Ltd. (Note 3) KF-6105: manufactured by Shin-Etsu Chemical Co., Ltd. | | | | | | |

The "←" in Table 6 means the contained amount was the same as in Example 4.

### <Preparation of cosmetics>

A: The components 1 to 14, 17, and 18 were heated and uniformly mixed.
B: Under heating, the components 15 and 16 were added to A and uniformly mixed, and the mixture was loaded into a prescribed highly airtight container to obtain a lipstick.

### [Usability evaluation]

Spreading at the time of application (spreadability), sticky feeling, finished color unevenness, and cosmetic durability (durability (evaluation 8 hours after application)) of the resulting lipsticks were evaluated by 50 female expert panelists in the same manner as the Examples and the evaluation results were averaged. Table 7 shows the results.

**[Table 7]**

| Items | Example 4 | Example 5 | Comparative Example 5 | Comparative Example 6 | Comparative Example 7 |
|---|---|---|---|---|---|
| Spreadability | Good | Good | Good | Fair | Good |
| Sticky feeling | Excellent | Excellent | Good | Excellent | Good |
| Color unevenness | Good | Excellent | Good | Poor | Good |
| Cosmetic durability | Excellent | Excellent | Good | Excellent | Good |

As shown in Table 7, it was confirmed that the lipsticks obtained in Examples 4 and 5 were not sticky and not oily, were free from bleeding, etc., and were also good in cosmetic durability compared with the lipsticks obtained in Comparative Examples 5, 6, and 7.

### [Examples 6 to 38]

Hereinafter, further formulation examples of cosmetics will be shown. Hereinafter, "spreadability" and "cosmetic durability" were evaluated according to the same criteria as described above, and with regard to foundations, secondary adhesion prevention was also evaluated according to the same criteria as described above.

### [Example 6] Powder foundation

| Composition | Mass (%) |
|---|---|
| 1. Silicone-treated titanium oxide (Note 1) | 12.0 |
| 2. Silicone-treated sericite (Note 1) | 35.0 |
| 3. Lecithin-treated talc | 35.1 |
| 4. Lecithin-treated spherical nylon powder | 5.0 |
| 5. Silicone-treated red oxide (Note 1) | 0.4 |
| 6. Silicone-treated yellow iron oxide (Note 1) | 2.0 |
| 7. Silicone-treated umber (Note 1) | 0.4 |
| 8. Silicone-treated black iron oxide (Note 1) | 0.1 |
| 9. Dissolved product of Synthesis Example 1 | 3.0 |
| 10. Crosslinked dimethylpolysiloxane (Note 2) | 4.0 |
| 11. Glyceryl trioctanoate | 1.5 |
| 12. Silicone wax (Note 3) | 1.5 |
| Total | 100.0 |

| | |
|---|---|
| (Note 1) KF-9909: manufactured by Shin-Etsu Chemical Co., Ltd., treatment (Note 2) KSG-16: manufactured by Shin-Etsu Chemical Co., Ltd. (Note 3) KP-562P: manufactured by Shin-Etsu Chemical Co., Ltd. | |

### <Preparation of cosmetics>

A: The components 1 to 8 were mixed, and the mixture was uniformly ground.
B: The components 9 to 12 were uniformly mixed.
C: B was added to A and homogenized, and the mixture was pressed into a mold to obtain a powder foundation.

The resulting powder foundation spread lightly, had good cosmetic durability and no secondary adhesion.

### [Example 7] Powder foundation

| Composition | Mass (%) |
|---|---|
| 1. Caprylylsilane-treated mica (Note 1) | 40.0 |
| 2. Silicone-treated talc (Note 2) | 20.0 |
| 3. Silicone-treated titanium oxide (Note 2) | 8.0 |
| 4. Silicone-treated fine particle titanium oxide (Note 2) | 5.0 |
| 5. Silicone-treated barium sulfate (Note 2) | 8.9 |
| 6. Silicone-treated foundation pigment (Note 2) | 7.0 |
| 7. Phenyl-modified hybrid silicone composite powder (Note 3) | 2.0 |
| 8. Polymethylsilsesquioxane powder (Note 4) | 0.4 |
| 9. Antiseptic | 0.5 |
| 10. Perfume | 0.2 |
| 11. Dissolved product of Synthesis Example 4 | 3.0 |
| 12. Glyceryl trioctanoate | 3.0 |
| 13. Squalane | 1.0 |
| 14. Vaseline | 1.0 |
| Total | 100.0 |

| | |
|---|---|
| (Note 1) AES-3083: manufactured by Shin-Etsu Chemical Co., Ltd., treatment (Note 2) KF-9909: manufactured by Shin-Etsu Chemical Co., Ltd., treatment (Note 3) KSP-300: manufactured by Shin-Etsu Chemical Co., Ltd. (Note 4) KMP-590: manufactured by Shin-Etsu Chemical Co., Ltd. | |

### <Preparation of cosmetics>

A: The components 1 to 9 were mixed, and the mixture was uniformly ground.
B: The components 11 to 14 were uniformly mixed, and the mixture was added to A to make them uniform.
C: The component 10 was added to B, and the mixture was pressed into a mold to obtain a powder foundation.

The resulting powder foundation spread lightly, had excellent cosmetic durability and no secondary adhesion.

### [Example 8] Stick W/O foundation

| Composition | Mass (%) |
|---|---|
| 1. Ceresin | 5.5 |
| 2. Inulin stearate | 2.0 |
| 3. Neopentyl glycol dioctanoate | 7.0 |
| 4. Triethylhexanoin | 4.0 |
| 5. Dimethylpolysiloxane (6cs) | 6.3 |
| 6. Dissolved product of Synthesis Example 2 | 3.0 |
| 7. Crosslinked polyglycerol-modified silicone (Note 1) | 4.0 |
| 8. Alkyl-modified branched polyglycerol-modified silicone (Note 2) | 1.5 |
| 9. Polymethylsilsesquioxane powder (Note 3) | 1.0 |
| 10. Silicone-treated titanium oxide (Note 4) | 9.0 |
| 11. Silicone-treated foundation pigment (Note 5) | 5.0 |
| 12. Lecithin | 0.2 |
| 13. Polysorbate 80 | 0.3 |
| 14. 1,3-BG | 4.0 |
| 15. Antiseptic | 0.5 |
| 16. Perfume | 0.2 |
| 17. Purified water | 46.5 |
| Total | 100.0 |

| | |
|---|---|
| (Note 1) KSG-710: manufactured by Shin-Etsu Chemical Co., Ltd. (Note 2) KF-6105: manufactured by Shin-Etsu Chemical Co., Ltd. (Note 3) KMP-590: manufactured by Shin-Etsu Chemical Co., Ltd. (Note 4) KF-9909: manufactured by Shin-Etsu Chemical Co., Ltd., treatment (Note 5) KF-9909: manufactured by Shin-Etsu Chemical Co., Ltd., treatment | |

### <Preparation of cosmetics>

A: The components 1 to 9 were heated, dissolved, and homogenized.
B: The components 10 to 13 and part of the component 14 were mixed, and the mixture was dispersed by a roller.
C: The remainder of the component 14, and the components 15 and 17 were uniformly dissolved, the solution was added to B, and under heating, the mixture was uniformly dispersed.
D: Under heating and stirring, C was added to A and emulsified, the component 16 was added to the emulsion, and the mixture was loaded into a prescribed highly airtight container to obtain a stick W/O foundation.

The resulting stick W/O foundation spread lightly, had excellent cosmetic durability and no secondary adhesion.

### [Example 9] Polyhydric alcohol-in-oil emulsion solid rouge

| Composition | Mass (%) |
|---|---|
| 1. Crosslinked polyglycerol-modified silicone (Note 1) | 5.0 |
| 2. Crosslinked dimethylpolysiloxane (Note 2) | 5.0 |
| 3. Decamethylcyclopentasiloxane | 3.0 |
| 4. Dimethylpolysiloxane (6cs) | 19.7 |
| 5. Cetyl isooctanoate | 5.0 |
| 6. Dissolved product of Synthesis Example 3 | 10.0 |
| 7. Behenyl-modified acrylic silicone resin (Note 3) | 3.0 |
| 8. Paraffin wax (Melting point 80°C) | 9.0 |
| 9. Dimethyl distearyl ammonium hectorite | 0.3 |
| 10. Acrylic silicone-treated powder (Note 4) | 25.0 |
| 11. Antiseptic | 0.5 |
| 12. Perfume | 0.2 |
| 13. 1,3-butylene glycol | 14.3 |
| Total | 100.0 |

| | |
|---|---|
| (Note 1) KSG-710: manufactured by Shin-Etsu Chemical Co., Ltd. (Note 2) KSG-15: manufactured by Shin-Etsu Chemical Co., Ltd. (Note 3) KP-562P: manufactured by Shin-Etsu Chemical Co., Ltd. (Note 4) KP-574: manufactured by Shin-Etsu Chemical Co., Ltd., treatment | |

### <Preparation of cosmetics>

A: The components 1 to 9 and 12 were heated to 80°C and uniformly mixed.
B: The component 10 was added to A and uniformly dispersed.
C: The components 11 and 13 were mixed and heated to 80°C, and then the mixture was added to B and emulsified, poured into a gold plate and cooled to obtain a polyhydric alcohol-in-oil emulsion solid rouge.

The polyhydric alcohol-in-oil emulsion solid rouge obtained as described above was light in the spreadability, and also not sticky and not oily.

### [Example 10] Cream lipstick

| Composition | Mass (%) |
|---|---|
| 1. Palmitic acid / ethyl hexanoic acid dextrin (Note 1) | 9.0 |
| 2. Triethylhexanoin | 7.0 |
| 3. Dissolved product of Synthesis Example 2 | 8.0 |
| 4. Alkyl-modified crosslinked dimethylpolysiloxane (Note 2) | 8.0 |
| 5. Alkyl-modified branched polyglycerol-modified silicone (Note 3) | 2.0 |
| 6. Decamethylcyclopentasiloxane | 35.0 |
| 7. 1,3-butylene glycol | 4.8 |
| 8. Purified water | 18.0 |
| 9. Coloring pigment | 6.0 |
| 10. Mica | 2.0 |
| 11. Perfume | 0.2 |
| Total | 100.0 |

| | |
|---|---|
| (Note 1) Rheopearl TT: manufactured by Chiba Flour Milling Co., Ltd. (Note 2) KSG-43: manufactured by Shin-Etsu Chemical Co., Ltd. (Note 3) KF-6105: manufactured by Shin-Etsu Chemical Co., Ltd. | |

### <Preparation of cosmetics>

A: The component 1, part of the component 2, and the components 3 to 6 were heated and uniformly mixed.
B: The component 9 was mixed with the remainder of the component 2 and dispersed by a roller, and the dispersion was added to A and uniformly mixed.
C: The components 7 and 8 were mixed, heated, and added to B, and the mixture was emulsified.
D: The components 10 and 11 were added to C to obtain a cream lipstick.

The cream lipstick obtained as described above was light in the spreadability, easy to spread on the lip, also not sticky and not oily, and also good in the durability.

### [Example 11] Eyeliner

| Composition | Mass (%) |
|---|---|
| 1. Tristrimethylsiloxy methylsilane (Note 1) | 20.0 |
| 2. Polyether-modified silicone (Note 2) | 3.0 |
| 3. Dissolved product of Synthesis Example 2 | 33.5 |
| 4. Silicone network resin dissolved product (Note 3) | 15.0 |
| 5. Dimethyl distearyl ammonium hectorite | 3.0 |
| 6. Silicone-treated black iron oxide (Note 4) | 10.0 |
| 7. 1,3-butylene glycol | 4.5 |
| 8. Sodium sulfate | 0.5 |
| 9. Antiseptic | 0.5 |
| 10. Purified water | 10.0 |
| Total | 100.0 |

| | |
|---|---|
| (Note 1) TMF-1.5: manufactured by Shin-Etsu Chemical Co., Ltd. (Note 2) KF-6017: manufactured by Shin-Etsu Chemical Co., Ltd. (Note 3) KF-7312T: manufactured by Shin-Etsu Chemical Co., Ltd. (Note 4) KF-9901: manufactured by Shin-Etsu Chemical Co., Ltd., treatment | |

### <Preparation of cosmetics>

A: The components 1 to 5 were mixed, and the component 6 was added to the mixture, and uniformly mixed and dispersed.
B: The components 7 to 10 were mixed.
C: B was added to A and emulsified to obtain an eyeliner.

It was confirmed that the eyeliner obtained as described above was light in the spreadability and easy to draw around the eyes, had the refreshing feeling in use, and also had very good cosmetic durability.

### [Example 12] Mascara

| Composition | Mass (%) |
|---|---|
| 1. Dissolved product of Synthesis Example 1 | 26.5 |
| 2. Dextrin palmitate/ethylhexanoate (Note 1) | 3.0 |
| 3. Ceresin | 2.5 |
| 4. Behenyl-modified acrylic silicone resin (Note 2) | 2.0 |
| 5. Bees wax | 3.5 |
| 6. Triethylhexanoin | 3.0 |
| 7. Dimethyl distearyl ammonium hectorite | 4.0 |
| 8. Lecithin | 0.5 |
| 9. Isododecane | 34.0 |
| 10. Silicone-treated pigment (Note 3) | 5.0 |
| 11. Silica | 3.0 |
| 12. Talc | 12.0 |
| 13. Branched polyether-modified silicone (Note 4) | 1.0 |
| Total | 100.0 |

| | |
|---|---|
| (Note 1) Rheopearl TT: manufactured by Chiba Flour Milling Co., Ltd. (Note 2) KP-562P: manufactured by Shin-Etsu Chemical Co., Ltd. (Note 3) KF-9909: manufactured by Shin-Etsu Chemical Co., Ltd., treatment (Note 4) KF-6028P: manufactured by Shin-Etsu Chemical Co., Ltd. | |

### <Preparation of cosmetics>

A: The components 7 and 13 were added to the component 9, heated, and uniformly mixed.
B: The components 1 to 6 and 8 were added to A, and uniformly mixed.
C: The components 10, 11, and 12 were added to B, and made uniform with a roller to obtain a mascara.

It was confirmed that the mascara obtained as described above was light in the spreadability, easily adhered to the eyelashes, had no sticky feeling in use, and also had very good cosmetic durability.

### [Example 13] Cream eye shadow

| Composition | Mass (%) |
|---|---|
| 1. Decamethylcyclopentasiloxane | 15.0 |
| 2. Dimethylpolysiloxane (6cs) | 4.0 |
| 3. Dissolved product of Synthesis Example 3 | 5.0 |
| 4. Branched polyether-modified silicone (Note 1) | 1.5 |
| 5. Acrylic silicone resin-treated pigment (Note 2) | 16.0 |
| 6. Sodium chloride | 2.0 |
| 7. Propylene glycol | 7.5 |
| 8. Antiseptic | 0.5 |
| 9. Purified water | 48.5 |
| Total | 100.0 |

| | |
|---|---|
| (Note 1) KF-6028P: manufactured by Shin-Etsu Chemical Co., Ltd. (Note 2) KP-574: manufactured by Shin-Etsu Chemical Co., Ltd., treatment | |

### <Preparation of cosmetics>

A: The components 1 to 4 were mixed, and the component 5 was added to the mixture, and uniformly mixed and dispersed.
B: The components 6 to 9 were mixed.
C: B was added to A and emulsified to obtain a cream eye shadow.

The cream eye shadow obtained as described above was light in the spreadability, not oily and not powdery, and also good in cosmetic durability.

### [Example 14] Cream eye shadow

| Composition | Mass (%) |
|---|---|
| 1. Acrylic silicone resin dissolved product (Note 1) | 3. 0 |
| 2. Stearyl-modified acrylic silicone resin (Note 2) | 2.0 |
| 3. Branched polyether-modified silicone (Note 3) | 1.5 |
| 4. Decamethylcyclopentasiloxane | 20.3 |
| 5. Dissolved product of Synthesis Example 4 | 10.0 |
| 6. Dimethyl distearyl ammonium hectorite | 1.2 |
| 7. Acrylic silicone resin-treated pigment (Note 4) | 20.0 |
| 8. Spherical nylon | 3.0 |
| 9. Talc | 4.0 |
| 10. Ethanol | 5.0 |
| 11. Purified water | 30.0 |
| Total | 100.0 |

| | |
|---|---|
| (Note 1) KP-545: manufactured by Shin-Etsu Chemical Co., Ltd. (Note 2) KP-561P: manufactured by Shin-Etsu Chemical Co., Ltd. (Note 3) KF-6028P: manufactured by Shin-Etsu Chemical Co., Ltd. (Note 4) KP-574: manufactured by Shin-Etsu Chemical Co., Ltd., treatment | |

### <Preparation of cosmetics>

A: The components 1 to 6 were mixed, and the components 7 to 9 were added to the mixture, and uniformly mixed and dispersed.
B: The components 10 and 11 were mixed.
C: B was added to A and emulsified to obtain a cream eye shadow.

The cream eye shadow obtained as described above was light in the spreadability, not oily and not powdery, was fresh, and also good in cosmetic durability.

### [Example 15] Sun-cut milky lotion

| Composition | Mass (%) |
|---|---|
| 1. Crosslinked polyether-modified silicone (Note | 1) 3.0 |
| 2. Crosslinked dimethylpolysiloxane (Note 2) | 2.0 |
| 3. Branched polyether-modified silicone (Note 3) | 1.0 |
| 4. Dissolved product of Synthesis Example 1 | 5.0 |
| 5. Decamethylcyclopentasiloxane | 5.0 |
| 6. Isotridecyl isononanoate | 4.0 |
| 7. Titanium oxide dispersion (Note 4) | 25.0 |
| 8. Zinc oxide dispersion (Note 5) | 35.0 |
| 9. 1,3-butylene glycol | 2.0 |
| 10. Sodium citrate | 0.2 |
| 11. Sodium chloride | 0.5 |
| 12. Purified water | 17.3 |
| Total | 100.0 |

| | |
|---|---|
| (Note 1) KSG-210: manufactured by Shin-Etsu Chemical Co., Ltd. (Note 2) KSG-15: manufactured by Shin-Etsu Chemical Co., Ltd. (Note 3) KF-6028P: manufactured by Shin-Etsu Chemical Co., Ltd. (Note 4) SPD-T5: manufactured by Shin-Etsu Chemical Co., Ltd. (Note 5) SPD-Z5: manufactured by Shin-Etsu Chemical Co., Ltd. | |

### <Preparation of cosmetics>

A: The components 1 to 6 were uniformly mixed.
B: The components 9 to 12 were mixed.
C: B was added to A and emulsified, the components 7 and 8 were added to the emulsion to obtain a sun-cut milky lotion.

The sun-cut milky lotion obtained as described above was light in the spreadability, not sticky and not oily, and good in perspiration resistance.

### [Example 16] Sun-cut cream

| Composition | Mass (%) |
|---|---|
| 1. Crosslinked polyether-modified silicone (Note 1) | 3.0 |
| 2. Crosslinked dimethylpolysiloxane (Note 2) | 2.0 |
| 3. Alkyl-modified branched polyether-modified silicone (Note 3) | 1.0 |
| 4. Dissolved product of Synthesis Example 3 | 7.0 |
| 5. Decamethylcyclopentasiloxane | 15.5 |
| 6. Octyl methoxycinnamate | 6.0 |
| 7. Acrylic silicone resin dissolved product (Note 4) | 10.0 |
| 8. Lipophilizing-treated fine particle zinc oxide (Note 5) | 20.0 |
| 9. 1,3-butylene glycol | 1.8 |
| 10. Sodium citrate | 0.2 |
| 11. Sodium chloride | 0.5 |
| 12. Perfume | 0.2 |
| 13. Purified water | 32.8 |
| Total | 100.0 |

| | |
|---|---|
| (Note 1) KSG-240: manufactured by Shin-Etsu Chemical Co., Ltd. (Note 2) KSG-15: manufactured by Shin-Etsu Chemical Co., Ltd. (Note 3) KF-6038: manufactured by Shin-Etsu Chemical Co., Ltd. (Note 4) KP-575: manufactured by Shin-Etsu Chemical Co., Ltd. (Note 5) AES-3083: manufactured by Shin-Etsu Chemical Co., Ltd., treatment | |

### <Preparation of cosmetics>

A: The component 7 was added to part of the component 5 and homogenized, and the component 8 was added to the mixture and dispersed by a beads mill.
B: The components 1 to 4, the remainder of the component 5, and the component 6 were uniformly mixed.
C: The components 9 to 11 and the component 13 were mixed, and homogenized.
D: C was added to B and emulsified, and A and the component 12 were added to the emulsion to obtain a sun-cut cream.

The sun-cut cream obtained as described above was not sticky, was light in the spreadability, not oily, gave a refreshing feeling in use, and was also good in cosmetic durability.

### [Example 17] Sun-cut lotion (shaking type)

| Composition | Mass (%) |
|---|---|
| 1. Branched polyether-modified silicone (Note 1) | 2.0 |
| 2. Dissolved product of Synthesis Example 2 | 5.0 |
| 3. Dimethylpolysiloxane (6cs) | 3.0 |
| 4. Decamethylcyclopentasiloxane | 7.8 |
| 5. Ethyl hexyl methoxycinnamate | 7.5 |
| 6. Hybrid silicone composite powder (Note 2) | 0.5 |
| 7. Dimethyl distearyl ammonium hectorite | 0.2 |
| 8. Zinc oxide dispersion (Note 3) | 45.0 |
| 9. 1,3-butylene glycol | 3.0 |
| 10. Alcohol | 5.0 |
| 11. Sodium citrate | 0.2 |
| 12. Sodium chloride | 0.5 |
| 13. Purified water | 20.3 |
| Total | 100.0 |

| | |
|---|---|
| (Note 1) KF-6028P: manufactured by Shin-Etsu Chemical Co., Ltd. (Note 2) KSP-105: manufactured by Shin-Etsu Chemical Co., Ltd. (Note 3) SPD-Z6: manufactured by Shin-Etsu Chemical Co., Ltd. | |

### <Preparation of cosmetics>

A: The components 1 to 7 were uniformly mixed.
B: The components 9 to 13 were mixed.
C: B was added to A and emulsified, and the component 8 was added to the emulsion to obtain a shaking type sun-cut lotion.

The sun-cut lotion obtained as described above was light in the spreadability, not sticky and not oily, and was also very excellent in cosmetic durability.

### [Example 18] Suntan milky lotion

| Composition | Mass (%) |
|---|---|
| 1. Crosslinked polyether-modified silicone (Note | 1) 2.0 |
| 2. Crosslinked dimethylpolysiloxane (Note 2) | 3.0 |
| 3. Polyether-modified silicone (Note 3) | 1.5 |
| 4. Dissolved product of Synthesis Example 1 | 2.0 |
| 5. Dimethylpolysiloxane (6cs) | 10.0 |
| 6. Decamethylcyclopentasiloxane | 15.3 |
| 7. Dihydroxyacetone | 2.0 |
| 8. Glycerol | 10.0 |
| 9. 1,3-butylene glycol | 5.0 |
| 10. Sodium citrate | 0.2 |
| 11. Sodium chloride | 0.5 |
| 12. Antioxidant | 0.5 |
| 13. Antiseptic | 0.5 |
| 14. Perfume | 0.2 |
| 15. Purified water | 47.3 |
| Total | 100.0 |

| | |
|---|---|
| (Note 1) KSG-210: manufactured by Shin-Etsu Chemical Co., Ltd. (Note 2) KSG-15: manufactured by Shin-Etsu Chemical Co., Ltd. (Note 3) KF-6017: manufactured by Shin-Etsu Chemical Co., Ltd. | |

### <Preparation of cosmetics>

A: The components 1 to 6 were uniformly mixed.
B: The components 7 to 13 and component 15 were mixed.
C: B was added to A and emulsified, and the component 14 was added to the emulsion to obtain a suntan milky lotion.

The suntan milky lotion obtained as described above was not sticky, was light in the spreadability, not oily, and gave a refreshing feeling in use.

### [Example 19] Suntan cream

| Composition | Mass (%) |
|---|---|
| 1. Alkyl-modified crosslinked polyether-modified silicone (Note 1) | 4.0 |
| 2. Alkyl-modified crosslinked dimethylpolysiloxane (Note 2) | 2.0 |
| 3. Alkyl-modified branched polyether-modified silicone (Note 3) | 1.0 |
| 4. Dissolved product of Synthesis Example 3 | 5.0 |
| 5. Decamethylcyclopentasiloxane | 10.3 |
| 6. Stearyl-modified acrylic silicone (Note 4) | 1.0 |
| 7. Dimethyl octyl para aminobenzoic acid | 1.5 |
| 8. 4-t-Butyl-4'-methoxy-dibenzoylmethane | 1.5 |
| 9. Kaolin | 0.5 |
| 10. Pigment | 8.0 |
| 11. Titanium oxide coated mica | 8.0 |
| 12. Dioctadecyl dimethyl ammonium chloride | 0.1 |
| 13. Sodium L-glutamate | 3.0 |
| 14. 1,3-butylene glycol | 4.0 |
| 15. Sodium citrate | 0.2 |
| 16. Sodium chloride | 0.5 |
| 17. Antioxidant | 0.5 |
| 18. Antiseptic | 0.5 |
| 19. Perfume | 0.2 |
| 20. Purified water | 48.2 |
| Total | 100.0 |

| | |
|---|---|
| (Note 1) KSG-320: manufactured by Shin-Etsu Chemical Co., Ltd. (Note 2) KSG-42: manufactured by Shin-Etsu Chemical Co., Ltd. (Note 3) KF-6038: manufactured by Shin-Etsu Chemical Co., Ltd. (Note 4) KP-561P: manufactured by Shin-Etsu Chemical Co., Ltd. | |

### <Preparation of cosmetics>

A: The components 1 to 8, 17 and 18 were heated and mixed.
B: The component 12 and part of the component 20 were heated and stirred, and then the components 9 to 11 were added to the mixture and dispersed.
C: The components 13 to 16 and the remainder of the component 20 were uniformly dissolved, and mixed with B. D: Under stirring, C was gradually added to A and emulsified, and cooled, and the component 19 was added to the emulsion to obtain a suntan cream.

The suntan cream obtained as described above had a fine texture, was light in the spreadability, not sticky and not oily, gave the refreshing feeling in use, and was also good in cosmetic durability.

### [Example 20] Liquid W/O foundation

| Composition | Mass (%) |
|---|---|
| 1. Decamethylcyclopentasiloxane | 18.0 |
| 2. Dimethylpolysiloxane (6cs) | 2.0 |
| 3. Dissolved product of Synthesis Example 1 | 7.0 |
| 4. Alkyl-modified branched polyether-modified silicone (Note 1) | 2.0 |
| 5. Ethyl hexyl paramethoxycinnamate | 3.0 |
| 6. Fluorine-modified silicone (Note 2) | 2.0 |
| 7. Polymethylsilsesquioxane powder (Note 3) | 1.5 |
| 8. Fluorine compound-treated foundation pigment (Note 4) | 9.3 |
| 9. Fluorine compound-treated mica titanium (Note 4) | 2.0 |
| 10. Silicone-treated fine particle titanium oxide (Note 5) | 8.0 |
| 11. Alkyl-modified branched polyglycerol-modified silicone (Note 6) | 1.2 |
| 12. Ethanol | 3.0 |
| 13. 1,3-butylene glycol | 4.3 |
| 14. Glycerol | 1.5 |
| 15. Magnesium sulfate | 0.5 |
| 16. Antioxidant | 0.5 |
| 17. Antiseptic | 0.5 |
| 18. Perfume | 0.2 |
| 19. Purified water | 33.5 |
| Total | 100.0 |

| | |
|---|---|
| (Note 1) KF-6038: manufactured by Shin-Etsu Chemical Co., Ltd. (Note 2) FL-5: manufactured by Shin-Etsu Chemical Co., Ltd. (Note 3) KMP-590: manufactured by Shin-Etsu Chemical Co., Ltd. (Note 4) 5% coated with perfluoroalkylethyl phosphate diethanolamine salt (Note 5) KF-9909: manufactured by Shin-Etsu Chemical Co., Ltd., treatment (Note 6) KF-6105: manufactured by Shin-Etsu Chemical Co., Ltd. | |

### <Preparation of cosmetics>

A: Part of the component 1 and the components 11 and 12 were mixed, and uniformly dispersed.
B: The components 8 to 10 were uniformly mixed.
C: The remainder of the component 1 and the components 2 to 7 were mixed, and B was added to the mixture, and uniformly mixed and dispersed.
D: The components 13 to 18 were mixed and homogenized.
E: Under stirring, D was gradually added to C and emulsified, and A and the component 19 were added to the emulsion to obtain a liquid W/O foundation.

The liquid W/O foundation obtained as described above was not sticky and was smooth, was light in the spreadability, not oily, and also good in cosmetic durability, and also free from the secondary adhesion.

### [Example 21] Hair cream

| Composition | Mass (%) |
|---|---|
| 1. Decamethylcyclopentasiloxane | 16.0 |
| 2. Methylphenylpolysiloxane (Note 1) | 2.0 |
| 3. Dissolved product of Synthesis Example 1 | 4.0 |
| 4. Squalane | 5.0 |
| 5. Silicone network resin dissolved product (Note 2) | 2.0 |
| 6. Sorbitan sesquiisostearate | 1.5 |
| 7. Alkyl-modified branched polyether-modified silicone (Note 3) | 2.0 |
| 8. Sorbitol sodium sulfate | 2.0 |
| 9. Chondroitin sodium sulfate | 1.0 |
| 10. Sodium hyaluronate | 0.5 |
| 11. Propylene glycol | 2.3 |
| 12. Antiseptic | 1.5 |
| 13. Vitamin E acetate | 0.1 |
| 14. Antioxidant | 0.5 |
| 15. Perfume | 0.2 |
| 16. Purified water | 59.4 |
| Total | 100.0 |

| | |
|---|---|
| (Note 1) KF-54: manufactured by Shin-Etsu Chemical Co., Ltd. (Note 2) KF-7312J: manufactured by Shin-Etsu Chemical Co., Ltd. (Note 3) KF-6038: manufactured by Shin-Etsu Chemical Co., Ltd. | |

### <Preparation of cosmetics>

A: The components 1 to 7 and the components 12 to 14 were uniformly mixed.
B: The components 8 to 11, and 16 were uniformly mixed.
C: Under stirring, B was gradually added to A and emulsified, and the component 15 was added to the emulsion to obtain a hair cream.

The hair cream obtained as described above was not oily, was light in the spreadability, was water resistant, water repellent, perspiration resistant, and also good in cosmetic durability.

### [Example 22] Hair cream

| Composition | Mass (%) |
|---|---|
| 1. Silicone gum dissolved product (Note 1) | 10.0 |
| 2. Silicone network resin dissolved product (Note 2) | 10.0 |
| 3. Dissolved product of Synthesis Example 3 | 10.0 |
| 4. Glyceryl tri-2-ethylhexanoate | 5.0 |
| 5. Vaseline | 5.0 |
| 6. Stearic acid | 1.5 |
| 7. Cetyl alcohol | 0.5 |
| 8. Polyglyceryl monooleate | 1.5 |
| 9. Glyceryl monostearate | 1.5 |
| 10. Polyether-modified silicone (Note 3) | 0.5 |
| 11. 1,3-butylene glycol | 5.0 |
| 12. (Acrylates / alkyl acrylate (C10-30)) cross polymer (Note 4) | 0.3 |
| 13. Triethanolamine | 0.3 |
| 14. Antiseptic | 0.5 |
| 15. Perfume | 0.2 |
| 16. Purified water | 48.2 |
| Total | 100.0 |

| | |
|---|---|
| (Note 1) KF-9028: manufactured by Shin-Etsu Chemical Co., Ltd. (Note 2) KF-7312J: manufactured by Shin-Etsu Chemical Co., Ltd. (Note 3) KF-6011: manufactured by Shin-Etsu Chemical Co., Ltd. (Note 4) Pemulen TR-1: manufactured by Noveon International, Inc. | |

### <Preparation of cosmetics>

A: The components 1 to 10 and the component 14 were heated and dissolved.
B: The components 11 to 13 and 16 were mixed and heated.
C: Under stirring, A was gradually added to B and emulsified, cooled, and then the component 15 was added to the emulsion to obtain a hair cream.

The hair cream obtained as described above was light in the spreadability, gave the hair gloss and smoothness, had an excellent setting effect on the hair, was water resistant, perspiration resistant, and also good in cosmetic durability.

### [Example 23] Moisturizing O/W cream

| Composition | Mass (%) |
|---|---|
| 1. Dissolved product of Synthesis Example 1 | 4.0 |
| 2. Liquid paraffin | 4.5 |
| 3. Macadamia nut oil | 5.0 |
| 4. Dimethylpolysiloxane (viscosity 6mm²/s:25°C) | 5.0 |
| 5. Octyl paramethoxycinnamate | 5.0 |
| 6. Alkyl-modified branched polyglycerol-modified silicone (Note 1) | 1.5 |
| 7. Propylene glycol | 8.0 |
| 8. Glycerol | 3.0 |
| 9. Antiseptic | 0.5 |
| 10. Perfume | 0.2 |
| 11. Purified water | 63.3 |
| Total | 100.0 |

| | |
|---|---|
| (Note 1) KF-6105: manufactured by Shin-Etsu Chemical Co., Ltd. | |

### <Preparation of cosmetics>

A: The components 1 to 6 were uniformly mixed.
B: The components 7 to 11 were mixed, and then added to A, and the mixture was emulsified to obtain a moisturizing O/W cream.

The moisturizing O/W cream obtained as described above was light in the spreadability, gave the refreshing feeling in use, and maintained a moisturizing effect.

### [Example 24] O/W emollient cream

| Composition | Mass (%) |
|---|---|
| 1. Crosslinked dimethylpolysiloxane (Note 1) | 7.0 |
| 2. Crosslinked dimethylpolysiloxane (Note 2) | 30.0 |
| 3. Dissolved product of Synthesis Example 2 | 6.0 |
| 4. Decamethylcyclopentasiloxane | 5.0 |
| 5. 1,3-butylene glycol | 4.0 |
| 6. Branched polyglycerol-modified silicone (Note | 3) 0.6 |
| 7. Branched polyglycerol-modified silicone (Note | 4) 0.3 |
| 8. (Acrylamide / Acryloyl dimethyl taurine Na) copolymer (Note 5) | 0.6 |
| 9. Dimethyl taurine ammonium acrylate / VP copolymer (Note 6) | 0.7 |
| 10. Sodium chloride | 0.1 |
| 11. Purified water | 45.7 |
| Total | 100.0 |

| | |
|---|---|
| (Note 1) KSG-15: manufactured by Shin-Etsu Chemical Co., Ltd. (Note 2) KSG-16: manufactured by Shin-Etsu Chemical Co., Ltd. (Note 3) KF-6104: manufactured by Shin-Etsu Chemical Co., Ltd. (Note 4) KF-6100: manufactured by Shin-Etsu Chemical Co., Ltd. (Note 5) Simulgel 600: manufactured by Seppic S.A. (Note 6) Aristoflex AVC: manufactured by Clariant AG | |

### <Preparation of cosmetics>

A: The components 1 to 4 were uniformly mixed.
B: The components 5 to 11 were uniformly mixed.
C: Under stirring, A was gradually added to B and mixed to obtain an O/W emollient cream.

The O/W emollient cream obtained as described above was not oily and was smooth, was light in the spreadability, and maintained a skin protection effect.

### [Example 25] Hand cream

| Composition | Mass (%) |
|---|---|
| 1. Decamethylcyclopentasiloxane | 25.0 |
| 2. Dissolved product of Synthesis Example 4 | 10.0 |
| 3. Liquid paraffin | 5.0 |
| 4. Amino-modified silicone gum dissolved product (Note 1) | 8.0 |
| 5. Branched polyether-modified silicone (Note 2) | 2.0 |
| 6. Hybrid silicone composite powder (Note 3) | 2.5 |
| 7. Distearyl dimethyl ammonium chloride | 0.8 |
| 8. Vitamin E acetate | 0.1 |
| 9. Polyethylene glycol 400 | 1.0 |
| 10. Glycerol | 10.0 |
| 11. Aluminum magnesium silicate | 1.2 |
| 12. Antiseptic | 0.5 |
| 13. Perfume | 0.2 |
| 14. Purified water | 33.7 |
| Total | 100.0 |

| | |
|---|---|
| (Note 1) KF-8108: manufactured by Shin-Etsu Chemical Co., Ltd. (Note 2) KF-6028P: manufactured by Shin-Etsu Chemical Co., Ltd. (Note 3) KSP-102: manufactured by Shin-Etsu Chemical Co., Ltd. | |

### <Preparation of cosmetics>

A: The components 1 to 8 and the component 12 were uniformly mixed.
B: The components 9 to 11, and 14 were uniformly mixed.
C: Under stirring, B was gradually added to A and emulsified, and the component 13 was added to the emulsion to obtain a hand cream.

The hand cream obtained as described above was not oily and was smooth, was light in the spreadability, and maintained the skin protection effect.

### [Example 26] O/W cream

| Composition | Mass (%) |
|---|---|
| 1. Dimethylpolysiloxane (6cs) | 7.0 |
| 2. Stearyl-modified acrylic silicone resin (Note 1) | 8.0 |
| 3. Dissolved product of Synthesis Example 3 | 5.0 |
| 4. Glyceryl triisostearate | 10.0 |
| 5. Cetanol | 1.0 |
| 6. Stearic acid | 3.0 |
| 7. Glyceryl monostearate | 1.5 |
| 8. Sorbitan sesquioleate | 0.5 |
| 9. Polyoxyethylene sorbitan monooleate | 1.0 |
| 10. Sodium hydroxide (1 mass% aqueous solution) | 10.0 |
| 11. 1,3-butylene glycol | 5.0 |
| 12. Antiseptic | 0.5 |
| 13. Perfume | 0.2 |
| 14. Purified water | 47.3 |
| Total | 100.0 |

| | |
|---|---|
| (Note 1) KP-561P: manufactured by Shin-Etsu Chemical Co., Ltd. | |

### <Preparation of cosmetics>

A: The components 1 to 9 were heated and mixed.
B: The components 10 to 12 and 14 were mixed and heated.
C: Under stirring, B was gradually added to A and emulsified and cooled, and then the component 13 was added to the emulsion to obtain an O/W cream.

It was confirmed that the O/W cream obtained as described above was not sticky and not oily, and was smooth, was light in the spreadability, and had the refreshing feeling in use.

### [Example 27] O/W cream

| Composition | Mass (%) |
|---|---|
| 1. Polyglyceryl monooleate | 1.0 |
| 2. Cetyl alcohol | 0.5 |
| 3. Stearic acid | 1.0 |
| 4. Glyceryl monostearate | 1.0 |
| 5. Dissolved product of Synthesis Example 1 | 2.0 |
| 6. Macadamian nut oil | 9.0 |
| 7. Crosslinked dimethylpolysiloxane (Note 1) | 0.5 |
| 8. (Acrylates / alkyl acrylate (C10-30)) cross polymer (Note 2) | 0.2 |
| 9. Methylcellulose | 0.1 |
| 10. Triethanolamine | 0.2 |
| 11. 1,3-butylene glycol | 7.0 |
| 12. Antiseptic | 0.5 |
| 13. Perfume | 0.2 |
| 14. Purified water | 76.8 |
| Total | 100.0 |

| | |
|---|---|
| (Note 1) KSG-16: manufactured by Shin-Etsu Chemical Co., Ltd. (Note 2) Pemulen TR-1: manufactured by Noveon International, Inc. | |

### <Preparation of cosmetics>

A: The components 1 to 7 were heated and uniformly mixed.
B: The components 8 to 12 and 14 were mixed and heated.
C: Under stirring, B was gradually added to A and emulsified and cooled, and then the component 13 was added to the emulsion to obtain an O/W cream.

It was confirmed that the O/W cream obtained as described above was not sticky and not oily, and was smooth, was light in the spreadability, and maintained freshness of the skin.

### [Example 28] Antiperspirant

| Composition | Mass (%) |
|---|---|
| 1. Crosslinked polyether-modified silicone (Note 1) | 7.0 |
| 2. Dissolved product of Synthesis Example 2 | 8.0 |
| 3. Decamethylcyclopentasiloxane | 9.0 |
| 4. 1,3-butylene glycol | 5.0 |
| 5. Sodium citrate | 0.2 |
| 6. Glycine salt of aluminum zirconium tetrachloride hydrate | 20.0 |
| 7. Purified water | 50.8 |
| Total | 100.0 |

| | |
|---|---|
| (Note 1) KSG-210: manufactured by Shin-Etsu Chemical Co., Ltd. | |

### <Preparation of cosmetics>

A: The components 1 to 3 were uniformly mixed.
B: The components 4 to 7 were uniformly mixed.
C: Under stirring, B was gradually added to A and emulsified to obtain an antiperspirant.

It was confirmed that the antiperspirant obtained as described above was light in the spreadability, did not whiten the skin, and had good duration of antiperspirant effect.

### [Example 29] Wrinkle concealer

| Composition | Mass (%) |
|---|---|
| 1. Crosslinked polyether-modified silicone (Note 1) | 5.0 |
| 2. Crosslinked dimethylpolysiloxane (Note 2) | 55.0 |
| 3. Dissolved product of Synthesis Example 1 | 15.0 |
| 4. Decamethylcyclopentasiloxane | 8.0 |
| 5. Hybrid silicone composite powder (Note 3) | 12.0 |
| 6. Silicone gum dissolved product (Note 4) | 5.0 |
| Total | 100.0 |

| | |
|---|---|
| (Note 1) KSG-210: manufactured by Shin-Etsu Chemical Co., Ltd. (Note 2) KSG-15: manufactured by Shin-Etsu Chemical Co., Ltd. (Note 3) KSP-101: manufactured by Shin-Etsu Chemical Co., Ltd. (Note 4) KF-9028: manufactured by Shin-Etsu Chemical Co., Ltd. | |

### <Preparation of cosmetics>

A: The components 1 to 6 were uniformly mixed to obtain a wrinkle concealer.

It was confirmed that the wrinkle concealer obtained as described above was not sticky and not oily, was smooth, was light in the spreadability, and was able to maintain a sealing effect.

### [Example 30] Cleansing cream

| Composition | Mass (%) |
|---|---|
| 1. Dimethylpolysiloxane (6cs) | 5.0 |
| 2. Methylphenylpolysiloxane (Note 1) | 5.0 |
| 3. Liquid paraffin | 5.0 |
| 4. Jojoba oil | 2.0 |
| 5. Dissolved product of Synthesis Example 4 | 4.0 |
| 6. Branched polyether-modified silicone (Note 2) | 2.0 |
| 7. Dextrin fatty acid ester | 0.8 |
| 8. Aluminum monostearate | 0.2 |
| 9. Aluminum chloride | 1.0 |
| 10. Glycerol | 10.0 |
| 11. Antiseptic | 0.5 |
| 12. Perfume | 0.2 |
| 13. Purified water | 64.3 |
| Total | 100.0 |

| | |
|---|---|
| (Note 1) KF-56: manufactured by Shin-Etsu Chemical Co., Ltd. (Note 2) KF-6028: manufactured by Shin-Etsu Chemical Co., Ltd. | |

### <Preparation of cosmetics>

A: The components 1 to 8 were heated and mixed.
B: The components 9 to 11 and 13 were heated and mixed.
C: Under stirring, B was gradually added to A and emulsified, and cooled, and the component 12 was added to the emulsion to obtain a cleansing cream.

It was confirmed that the cleansing cream obtained as described above was light in the spreadability, was moist and fresh, and gave the refreshing feeling in use.

### [Example 31] Transparent cleansing lotion

| Composition | Mass (%) |
|---|---|
| 1. Decamethylcyclopentasiloxane | 50.8 |
| 2. Dissolved product of Synthesis Example 1 | 5.0 |
| 3. Neopentyl glycol dioctanoate | 6.0 |
| 4. Silica | 0.2 |
| 5. 1,3-butylene glycol | 5.0 |
| 6. Glycerol | 6.0 |
| 7. Polyether-modified silicone (Note 1) | 5.0 |
| 8. Polyether-modified silicone (Note 2) | 3.0 |
| 9. PEG-60 hydrogenated castor oil | 2.0 |
| 10. Purified water | 17.0 |
| Total | 100.0 |

| | |
|---|---|
| (Note 1) KF-6011: manufactured by Shin-Etsu Chemical Co., Ltd. (Note 2) KF-6013: manufactured by Shin-Etsu Chemical Co., Ltd. | |

### <Preparation of cosmetics>

A: The components 1 to 4 were uniformly mixed.
B: The components 5 to 10 were uniformly mixed.
C: Under stirring, A was gradually added to B and emulsified to obtain a transparent cleansing lotion.

It was confirmed that the transparent cleansing lotion obtained as described above was light in the spreadability, gave the moist and fresh feeling in use, and also had a high cleansing effect.

### [Example 32] W/O rouge

| Composition | Mass (%) |
|---|---|
| 1. Acrylic silicone resin dissolved product (Note 1) | 10.0 |
| 2. Stearyl-modified acrylic silicone resin (Note 2) | 2.0 |
| 3. Branched polyether-modified silicone (Note 3) | 1.5 |
| 4. Decamethylcyclopentasiloxane | 15.0 |
| 5. Glyceryl triisostearate | 3.0 |
| 6. Dissolved product of Synthesis Example 2 | 5.0 |
| 7. Dimethyl distearyl ammonium hectorite | 1.5 |
| 8. Spherical nylon | 3.0 |
| 9. Talc | 4.0 |
| 10. Rouge pigment (Acrylic silicone-treated) (Note 4) | 20.0 |
| 11. Alcohol | 5.0 |
| 12. Perfume | 0.2 |
| 13. Purified water | 29.8 |
| Total | 100.0 |

| | |
|---|---|
| (Note 1) KP-545: manufactured by Shin-Etsu Chemical Co., Ltd. (Note 2) KP-561P: manufactured by Shin-Etsu Chemical Co., Ltd. (Note 3) KF-6028P: manufactured by Shin-Etsu Chemical Co., Ltd. (Note 4) KP-574: manufactured by Shin-Etsu Chemical Co., Ltd., treatment | |

### <Preparation of cosmetics>

A: The components 1 to 7 were warmed and mixed.
B: The components 8 to 10 and the component 12 were uniformly mixed, and mixed with A.
C: The components 11 and 13 were mixed.
D: Under stirring, C was gradually added to B and emulsified to obtain a W/O rouge.

The W/O rouge obtained as described above was not sticky and not oily, was light in the spreadability, excellent in adhesion, and also good in the cosmetic durability.

### [Example 33] W/O liquid foundation

| Composition | Mass (%) |
|---|---|
| 1. Crosslinked polyether-modified silicone (Note 1) | 3.0 |
| 2. Crosslinked dimethylpolysiloxane (Note 2) | 5.0 |
| 3. Branched polyether-modified silicone (Note 3) | 2.0 |
| 4. Decamethylcyclopentasiloxane | 20.0 |
| 5. Cetyl isooctanoate | 5.0 |
| 6. Dissolved product of Synthesis Example 2 | 10.0 |
| 7. Dimethyl distearyl ammonium hectorite | 1.2 |
| 8. Foundation pigment (silicone-treated) (Note 4) | 14.0 |
| 9. Acrylic silicone resin dissolved product (Note 5) | 10.0 |
| 10. 1,3-butylene glycol | 5.0 |
| 11. Xanthan gum | 0.1 |
| 12. Sodium citrate | 0.2 |
| 13. Sodium chloride | 0.5 |
| 14. Antiseptic | 0.5 |
| 15. Perfume | 0.2 |
| 16. Purified water | 23.3 |
| Total | 100.0 |

| | |
|---|---|
| (Note 1) KSG-210: manufactured by Shin-Etsu Chemical Co., Ltd. (Note 2) KSG-15: manufactured by Shin-Etsu Chemical Co., Ltd. (Note 3) KF-6028P: manufactured by Shin-Etsu Chemical Co., Ltd. (Note 4) KF-9909: manufactured by Shin-Etsu Chemical Co., Ltd., treatment (Note 5) KP-575: manufactured by Shin-Etsu Chemical Co., Ltd. | |

### <Preparation of cosmetics>

A: Part of the component 4 and the component 9 were mixed, and the component 8 was dispersed uniformly in the mixture.
B: The components 1 to 3, the remainder of the component 4, and the components 5 to 7 were uniformly mixed.
C: The components 10 to 14 and the component 16 were uniformly mixed.
D: Under stirring, C was gradually added to B and emulsified, and A and the component 15 were added to the emulsion to obtain a W/O liquid foundation.

The W/O liquid foundation obtained as described above was not sticky and not oily, was light in the spreadability, also good in the cosmetic durability, and also free from the secondary adhesion.

### [Example 34] W/O cream

| Composition | Mass (%) |
|---|---|
| 1. Crosslinked alkyl polyether-modified silicone (Note 1) | 3.0 |
| 2. Crosslinked alkyl-modified dimethylpolysiloxane (Note 2) | 4.0 |
| 3. Alkyl-modified branched polyether-modified silicone (Note 3) | 1.0 |
| 4. Meadowfoam oil | 3.5 |
| 5. Jojoba oil | 2.5 |
| 6. Macadamian nut oil | 5.0 |
| 7. Dissolved product of Synthesis Example 3 | 7.5 |
| 8. Hybrid silicone composite powder (Note 4) | 3.0 |
| 9. 1,3-butylene glycol | 8.0 |
| 10. Glycine | 3.0 |
| 11. Sodium citrate | 0.2 |
| 12. Sodium chloride | 0.5 |
| 13. Antiseptic | 0.5 |
| 14. Perfume | 0.2 |
| 15. Purified water | 58.1 |
| Total | 100.0 |

| | |
|---|---|
| (Note 1) KSG-340: manufactured by Shin-Etsu Chemical Co., Ltd. (Note 2) KSG-44: manufactured by Shin-Etsu Chemical Co., Ltd. (Note 3) KF-6038: manufactured by Shin-Etsu Chemical Co., Ltd. (Note 4) KSP-100: manufactured by Shin-Etsu Chemical Co., Ltd. | |

### <Preparation of cosmetics>

A: The components 1 to 8 were uniformly mixed.
B: The components 9 to 13 and the component 15 were uniformly mixed.
C: Under stirring, B was gradually added to A and emulsified, and the component 14 was added to the emulsion to obtain a W/O cream.

It was confirmed that the W/O cream obtained as described above was not sticky and not oily, and maintained a moist feeling of the skin.

### [Example 35] Cuticle coat

| Composition | Mass (%) |
|---|---|
| 1. Polyether-modified silicone (Note 1) | 3.0 |
| 2. Polyether-modified silicone (Note 2) | 2.0 |
| 3. PEG-40 hydrogenated hardened castor oil | 1.0 |
| 4. Dissolved product of Synthesis Example 4 | 3.0 |
| 5. Silicone gum dissolved product (Note 3) | 40.0 |
| 6. Decamethylcyclopentasiloxane | 40.0 |
| 7. Alcohol | 4.3 |
| 8. Antiseptic | 0.5 |
| 9. Perfume | 0.2 |
| 10. Purified water | 6.0 |
| Total | 100.0 |

| | |
|---|---|
| (Note 1) KF-6011: manufactured by Shin-Etsu Chemical Co., Ltd. (Note 2) KF-6013: manufactured by Shin-Etsu Chemical Co., Ltd. (Note 3) KF-9028: manufactured by Shin-Etsu Chemical Co., Ltd. | |

### <Preparation of cosmetics>

A: The components 1 to 3, and 7 to 10 were uniformly mixed.
B: The components 4 to 6 were uniformly mixed.
C: Under stirring, B was added to A and emulsified to obtain a cuticle coat.

It was confirmed that the cuticle coat obtained as described above was light in the spreadability, suppressed overly dry of the hair, and gave the hair gloss and smoothness.

### [Example 36] Hair treatment

| Composition | Mass (%) |
|---|---|
| 1. Silicone gum dissolved product (Note 1) | 5.0 |
| 2. Diphenyl dimethicone (Note 2) | 4.0 |
| 3. Dissolved product of Synthesis Example 4 | 1.0 |
| 4. Cetyl octanoate | 1.0 |
| 5. Cetyl alcohol | 0.5 |
| 6. Polyether-modified silicone (Note 3) | 1.0 |
| 7. PEG-60 hydrogenated hardened castor oil | 1.0 |
| 8. Glyceryl monostearate | 0.5 |
| 9. Carboxyvinyl polymer (1 mass% aqueous solution) | 25.0 |
| 10. Xanthan gum (1 mass% aqueous solution) | 7.0 |
| 11. 1,3-butylene glycol | 5.0 |
| 12. Alcohol | 7.0 |
| 13. Antiseptic | 0.5 |
| 14. Perfume | 0.2 |
| 15. Purified water | 41.3 |
| Total | 100.0 |

| | |
|---|---|
| (Note 1) MK-15H: manufactured by Shin-Etsu Chemical Co., Ltd. (Note 2) KF-54: manufactured by Shin-Etsu Chemical Co., Ltd. (Note 3) KF-6013: manufactured by Shin-Etsu Chemical Co., Ltd. | |

### <Preparation of cosmetics>

A: The components 1 to 8 were heated and dissolved.
B: The components 11 to 15 were heated and dissolved.
C: Under stirring, B was added to A and emulsified, and further the components 9 and 10 were added to the emulsion to obtain a hair treatment.

It was confirmed that the hair treatment obtained as described above was light in the spreadability and gave the hair gloss and smoothness.

### [Example 37] Nail enamel

| Composition | Mass (%) |
|---|---|
| 1. Dissolved product of Synthesis Example 2 | 35.0 |
| 2. Tristrimethylsiloxy methylsilane (Note 1) | 5.0 |
| 3. Nitrocellulose | 3.0 |
| 4. Camphor | 0.5 |
| 5. Acetyltributyl citrate | 1.0 |
| 6. Dimethyl distearyl ammonium hectorite | 0.5 |
| 7. Butyl acetate | 30.0 |
| 8. Ethyl acetate | 13.0 |
| 9. Isopropyl alcohol | 5.0 |
| 10. Coloring pigment | 7.0 |
| Total | 100.0 |

| | |
|---|---|
| (Note 1) TMF-1.5: manufactured by Shin-Etsu Chemical Co., Ltd. | |

### <Preparation of cosmetics>

A: The components 7 to 9 were mixed, and the components 4 to 6 were added to the mixture and uniformly mixed.
B: The components 1 to 3 were added to A and mixed.
C: The component 10 was added to B and mixed to obtain a nail enamel.

It was confirmed that the nail enamel obtained as described above was light in the spreadability, gave the nail gloss, and was also excellent in durability.

### [Example 38] Nail enamel overcoat

| Composition | Mass (%) |
|---|---|
| 1. Dissolved product of Synthesis Example 4 | 6.0 |
| 2. Nitrocellulose | 17.0 |
| 3. Alkyd resin | 4.0 |
| 4. Acetyltriethyl citrate | 5.0 |
| 5. Butyl acetate | 29.0 |
| 6. Ethyl acetate | 25.0 |
| 7. Isopropyl alcohol | 3.0 |
| 8. n-butyl alcohol | 1.0 |
| 9. Toluene | 10.0 |
| Total | 100.0 |

### <Preparation of cosmetics>

A: The components 5 to 9 were mixed, and the component 4 was added to the mixture and uniformly mixed.
B: The components 1 to 3 were added to A and mixed to obtain a nail enamel overcoat.

It was confirmed that the nail enamel overcoat obtained as described above was light in the spreadability, enhanced the gloss of the enamel, and also had good durability.

Conventionally, the use of a silicone resin that forms a hard, cracked film for a cosmetic that requires cosmetic durability and secondary adhesion prevention has not been considered. However, the inventive cosmetic, which contains a specific silicone resin, has no stickiness upon application, has smooth spreadability, excellent feeling on use, good water resistance, and excellent cosmetic durability owing to good adhesion to skin, can prevent adhesion to clothes or the like (secondary adhesion), and has excellent abrasion resistance as described above.

It should be noted that the present invention is not limited to the above-described embodiments. The embodiments are just examples, and any examples that have substantially the same feature and demonstrate the same functions and effects as those in the technical concept disclosed in claims of the present invention are included in the technical scope of the present invention.

## Claims

1. A cosmetic comprising a silicone resin whose constitutional units consist of the following general formulae (A), (B), (C), and (D) :
(A) (R¹₃SiO_{1/2})a;
(B) (R²₂SiO_{2/2})b;
(C) (R³SiO_{3/2})c;
and
(D) (SiO_{4/2})d,
wherein R¹, R², and R³ each independently represent a group selected from an alkyl group having 1 to 8 carbon atoms, an aryl group having 6 to 12 carbon atoms, and a fluorine-substituted alkyl group having 1 to 8 carbon atoms,
"a" has a value of 0.2 to 0.5,
"b" has a value of 0.0 to 0.1,
"c" has a value of 0.2 to 0.6,
"d" has a value of 0.2 to 0.5,
a+b+c+d has a value of 1.0, a/(c+d) has a value of 0.3 to 0.9, and c/d has a value of 0.4 to 3.0,
the silicone resin having a weight-average molecular weight of more than 20000 and 100000 or less.

2. The cosmetic according to claim 1, containing the silicone resin in an amount of 0.1 to 40 mass% based on a total mass of the cosmetic.

3. The cosmetic according to claim 1 or 2, further comprising an oil agent selected from a silicone oil, a hydrocarbon oil, an ester oil, and a glyceride oil.

4. The cosmetic according to any one of claims 1 to 3, further comprising a surfactant.

5. The cosmetic according to claim 4, wherein the surfactant is: a linear or branched organopolysiloxane whose hydrophilic group is polyoxyalkylene or polyglycerin; or an alkyl-co-modified organopolysiloxane thereof.

6. The cosmetic according to any one of claims 1 to 5, further comprising a composition composed of crosslinked organopolysiloxane polymer and a liquid oil agent.

7. The cosmetic according to claim 6, wherein the crosslinked organopolysiloxane polymer has a polyether group and/or a polyglycerin group.

8. The cosmetic according to any one of claims 1 to 7, further comprising a silicone film former selected from a group consisting of: other than the silicone resin, a silicone resin composed of components selected from R₃SiO_{1/2}, R₂SiO_{2/2}, RSiO_{3/2}, and SiO_{4/2}, wherein R is the same as the R¹; a linear acrylic-silicone graft copolymer; and a linear acrylic-silicone block copolymer.

9. The cosmetic according to any one of claims 1 to 8, further comprising a powder.

10. The cosmetic according to any one of claims 1 to 9, further comprising an ultraviolet light absorbing component.

11. The cosmetic according to any one of claims 1 to 10, wherein the cosmetic is one of a water-based, oilbased, or emulsion-based skin care cosmetic, hair cosmetic, antiperspirant, make-up cosmetic, or ultraviolet protection cosmetic.
